(19) Europäisches Patentamt
European Patent Office
Office européen des brevets

(11) EP 1 534 351 B1

# (12) EUROPEAN PATENT SPECIFICATION

(45) Date of publication and mention of the grant of the patent:
**04.10.2006 Bulletin 2006/40**

(21) Application number: **03785130.0**

(22) Date of filing: **08.08.2003**

(51) Int Cl.:
*A61L 24/04* (2006.01) *A61L 24/00* (2006.01)
*A61L 27/50* (2006.01) *A61L 27/16* (2006.01)
*A61L 31/04* (2006.01) *A61L 31/14* (2006.01)
*A61K 9/16* (2006.01)

(86) International application number:
**PCT/US2003/025017**

(87) International publication number:
**WO 2004/014446 (19.02.2004 Gazette 2004/08)**

## (54) EMBOLIZATION

EMBOLISATION

EMBOLISATION

(84) Designated Contracting States:
**DE FR GB IE NL**

(30) Priority: **09.08.2002 US 215594**
**08.07.2003 US 615276**

(43) Date of publication of application:
**01.06.2005 Bulletin 2005/22**

(73) Proprietor: **Boston Scientific Limited**
**St. Michael (BB)**

(72) Inventors:
- **LANPHERE, Janel**
  **Hyde Park, MA 02136 (US)**
- **ST. PIERRE, Ernest, J.**
  **South Attleboro, MA 02703 (US)**
- **KAPOGLIS, Greg**
  **Salem, MA 01970 (US)**
- **CASEY, Thomas, V., II**
  **Grafton, MA 01519 (US)**

(74) Representative: **Altenburg, Udo**
**Patent- und Rechtsanwälte**
**Bardehle . Pagenberg . Dost .**
**Altenburg - Geissler**
**Galileiplatz 1**
**81679 München (DE)**

(56) References cited:
**EP-A- 0 402 031** **EP-A- 0 730 847**
**WO-A-02/11696** **WO-A-93/19702**
**WO-A-96/39464** **DE-A- 10 026 620**
**US-A- 5 571 182** **US-A1- 2001 051 670**

- **DATABASE WPI Section Ch, Week 199808 Derwent Publications Ltd., London, GB; Class A14, AN 1998-082801 XP002250507 & JP 09 316271 A (KURARAY CO LTD), 9 December 1997 (1997-12-09)**
- **YUSI G M ET AL: "SUBMUCOSAL INJECTION OF POLYVINYL ALCOHOL IN ARTIFICIALLY CREATED VESICO-URETERAL REFLUX: A PRELIMINARY REPORT" ASIAN JOURNAL OF SURGERY, DEPARTMENT OF SURGERY, UNIVERSITY OF HONGKONG, HONG KONG, HK, vol. 18, no. 2, 1995, pages 122-127, XP008020559 ISSN: 1015-9584**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 1 534 351 B1

## Description

### TECHNICAL FIELD

The invention relates to embolization.

### BACKGROUND

Therapeutic vascular occlusions (embolizations) are used to prevent or treat pathological conditions *in situ.* Compositions including embolic particles are used for occluding vessels in a variety of medical applications. Delivery of embolic particles through a catheter is dependent on size uniformity, density and compressibility of the embolic particles.

DATABASE WPI Section Ch, Week 199808 Derwent Publications Ltd., London, GB; class A 14, AN 1998-082801 XP002250507 & JP 09 316271 A (KURARAY CO LTD) 9 December 1997 (1997-12-09) discloses spherical water containing gel particles, comprising acetylated polyvinyl alcohol which can be used in a carrier of a bio-catalyst, water preserver, substitute for bio-gels, a drug slow release agent and actuator substrate.

DE 100 26 620 A describes biocompatible material for cell and tissue implantation useful for drug release or cosmetic tissue augmentation, consisting of spherical particles having (semi-)penneable or porous outer shell and internal cavity.

US 2001/051670 A1 describes a composition for tissue bulking and coating comprising macromers which can be crosslinked to form a hydrogel in the form of a microsphere.

### SUMMARY

In one aspect, the invention features a polymeric particle having a diameter of about 500 microns or less. The particle has a first density of pores in an interior region and a second density of pores at a surface region. The first density is different from the second density.

In another aspect, the invention features a polymeric particle having a diameter of about 500 microns or less. The particle has a first average pore size in an interior region and a second average pore size at the surface region. The first average pore size is different from the second average pore size.

In a further aspect, the invention features a composition that includes a plurality of particles in a carrier fluid. At least some of the plurality of particles have a diameter of about 500 microns or less. At least some of the particles having a diameter of 500 microns or less have a first density of pores in an interior region and a second density of pores at a surface region. The first density is different from the second density.

In one aspect, the invention features a composition that includes a plurality of particles in a carrier fluid. At least some of the plurality of particles have a diameter of 500 microns or less. At least some of the particles having a diameter of 500 microns or less have a first average pore size in an interior region and a second average pore size at a surface region. The first average pore size is different from the second average pore size.

In another aspect, the invention features a method that includes passing a solution that contains a base polymer and a gelling precursor through an orifice having a diameter of 200 microns or less (e.g., 100 microns or less, 10 microns or more) to form drops containing the base polymer and the gelling precursor. The method also includes forming particles containing the base polymer and the gelling precursor from the drops containing the base polymer and the gelling precursor.

In a further aspect, the invention features a method that includes heating a solution that contains a base polymer and a gelling precursor to a temperature of at least 50˚C (e.g., 65˚C or more, 75˚C or more, 85˚C or more, 95˚C or more, 105˚C or more, 115˚C or more, 121˚C). The method also include forming particles containing the base polymer and the gelling precursor from the solution containing the base polymer and the gelling precursor.

In one aspect, the invention features a method that includes passing a solution containing a base polymer and a gelling precursor through an orifice while vibrating the orifice at a frequency of 0.1 KHz or more (e.g., 0.8 KHz or more, 1.5 KHz or more) to form drops containing the base polymer and the gelling precursor. The method also includes forming particles containing the base polymer and the gelling precursor from the drops containing the base polymer and the gelling precursor.

In another aspect, the invention features a method that includes forming drops containing the base polymer and the gelling precursor, and contacting the drops with a gelling agent to form particles containing the base polymer and the gelling precursor. The gelling agent is at a temperature greater than room temperature (e.g., a temperature of 30˚C or more).

In a further aspect, the invention features a method that includes forming drops containing a base polymer and a gelling precursor, and contacting the drops with a gelling agent to form particles containing the base polymer and the gelling precursor. The gelling agent is contained in a vessel, and the method further includes bubbling a gas through

the gelling agent, disposing a mist containing the gelling agent between a source of the drops and the vessel, including a surfactant in the mixture containing the gelling agent, and/or stirring the gelling agent.

In one aspect, the invention features a method that includes administering to a subject a therapeutically effective amount of a composition including a plurality of particles in a carrier fluid. At least some of the plurality of particles have a diameter of 500 microns or less. At least some of the particles having a diameter of 500 microns or less have a first density of pores in an interior region and a second density of pores at a surface region. The first density is different from the second density.

In another aspect, the invention features a method that includes administering to a subject a therapeutically effective amount of a composition including a plurality of particles in a carrier fluid. At least some of the plurality of particles have a diameter of 500 microns or less. At least some of the particles having a diameter of 500 microns or less have a first average pore size in an interior region and a second average pore size at a surface region. The first average pore size is different from the second average pore size.

Embodiments may also include one or more of the following.

The first density can be greater than the second density.

The first average pore size can be greater than the second average pore size.

A particle can have a diameter of 10 microns or more. A particle can have a diameter of 100 microns or more and/or a diameter of 300 microns or less. A particle can have a diameter of 300 microns or more.

A particle can include at least one polymer selected from polyvinyl alcohols, polyacrylic acids, polymethacrylic acids, poly vinyl sulfonates, carboxymethyl celluloses, hydroxyethyl celluloses, substituted celluloses, polyacrylamides, polyethylene glycols, polyamides, polyureas, polyurethanes, polyesters, polyethers, polystyrenes, polysaccharides, polylactic acids, polyethylenes, polymethylmethacrylates, polycaprolactones, polyglycolic acids, and poly(lactic-co-glycolic) acids.

A particle can be at least partially coated with a substantially bioabsorbable material.

A particle can have a density of from 1.1 grams per cubic centimeter to 1.4 grams per cubic centimeter.

A particle can have a sphericity of 0.9 or more.

After compression to about 50 percent, a particle has a sphericity of 0.9 or more.

A particle can include 2.5 weight percent or less polysaccharide (e.g., alginate). An alginate can have a guluronic acid content of about 60 percent or greater.

A particle can be substantially insoluble in DMSO.

A particle can be substantially free of animal-derived compounds.

A carrier fluid can include a saline solution, a contrast agent or both.

A plurality of particles can have a mean diameter of 500 microns or less and/or 10 microns or more. A plurality of particles can have a mean diameter of 100 microns or more and/or a mean diameter of 300 microns or less. A plurality of particles can have a mean diameter of 300 microns or more.

A method can include heating the solution to a temperature of at least 50˚C before passing the solution through the orifice.

A method can include vibrating the nozzle orifice at a frequency of at least 0.1 KHz as the solution passes therethrough.

A method can further include contacting the drops with a gelling agent to gel the gelling precursor to form particles comprising the base polymer and gelled gelling precursor.

A method can further include removing at least some of the gelled gelling precursor from the particles.

A composition can be administered by percutaneous injection.

A composition can be administered by a catheter.

A composition can be introduced into the subject using a lumen having a diameter that is smaller than a mean diameter of the plurality of particles.

A composition can be used to treat a cancer condition. The cancer condition can be, for example, ovarian cancer, colorectal cancer, thyroid cancer, gastrointestinal cancer, breast cancer, prostate cancer and/or lung cancer. Treating the cancer condition can include at least partially occluding a lumen providing nutrients to a site of the cancer condition with at least some of the plurality of particles.

A method can include at least partially occluding a lumen in the subject with at least some of a plurality of particles.

Embodiments of the invention may have one or more of the following advantages. Some disorders or physiological conditions can be mediated by delivery of embolic compositions. Embolic compositions can be used, for example, in treatment of fibroids, tumors (e.g., hypervascular tumors), internal bleeding, and/or arteriovenous malformations (AVMs). Examples of fibroids can include uterine fibroids which grow within the uterine wall, on the outside of the uterus, inside the uterine cavity, between the layers of broad ligament supporting the uterus, attached to another organ or on a mushroom-like stalk. Internal bleeding includes gastrointestinal, urinary, renal and varicose bleeding. AVMs are, for example, abnormal collections of blood vessels which shunt blood from a high pressure artery to a low pressure vein. The result can be hypoxia and malnutrition of those regions from which the blood is diverted.

Spherical embolic particles in the embolic compositions can be tailored to a particular application by, for example, varying particle size, porosity gradient, compressibility, sphericity and density of the particles. In embodiments in which the spherical embolic particles have a substantially uniform size, the particles can, for example, fit through the aperture of a catheter for administration by injection to a target site, without partially or completely plugging the lumen of the catheter. The spherical embolic particles have a mean diameter of 1200 microns or less (e.g., from 100 microns to 500 microns). Size uniformity of ± 15 percent of the spherical embolic particles allows the particles to stack evenly in the cylindrical lumen of the blood vessel to completely occlude the blood vessel lumen. Suspensions containing the embolic particles at a density of 1.1 grams per cubic centimeter to 1.4 grams per cubic centimeter can be prepared in calibrated concentrations of the embolic particles for ease of delivery by the physician without rapid settlement of the suspension. Control in sphericity and uniformity of the embolic particles can result in reduction in aggregation caused, for example, by surface interaction of the particles. In addition, the embolic particles are relatively inert in nature.
Features and advantages are in the description, drawings, and claims.

## DESCRIPTION OF DRAWINGS

**FIG. 1A** is a schematic illustrating injection of an embolic composition including embolic particles into a vessel, while **FIG. 1B** is an enlarged view of the region 1B in **FIG. 1A;**
**FIG. 2A** is a light micrograph of a collection of hydrated embolic particles, while **FIG. 2B** is a scanning electron microscope (SEM) photograph of an embolic particle surface and FIGS. **2C-2E** are cross-sections of embolic particles;
**FIG. 3A** is a schematic of the manufacture of an embolic composition while **FIG. 3B** is an enlarged schematic of region 3B in **FIG. 3A;**
**FIG. 4** is a photograph of gel-stabilized drops;
**FIG. 5** is a graph of embolic particle size uniformity;
**FIG. 6** is a graph of embolic particle size uniformity;
**FIG. 7** is a schematic of an injection pressure testing equipment;
**FIG. 8** is an infrared spectrum of embolic particles; and
**FIG. 9** is an infrared spectrum of embolic particles.

## DETAILED DESCRIPTION

### Composition

Referring to **FIGS. 1A** and **1B,** an embolic composition, including embolic particles **111** and a carrier fluid, is injected into a vessel through an instrument such as a catheter **150.** Catheter **150** is connected to a syringe barrel **110** with a plunger **160.** Catheter **150** is inserted, for example, into a femoral artery **120** of a patient. Catheter **150** delivers the embolic composition to, for example, occlude a uterine artery **130** leading to a fibroid **140.** Fibroid **140** is located in the uterus of a female patient. The embolic composition is initially loaded into syringe **110.** Plunger **160** of syringe **110** is then compressed to deliver the embolic composition through catheter **150** into a lumen **165** of uterine artery **130.**
Referring particularly to **FIG. 1B** which is an enlarged view of section 1B of **FIG. 1A,** uterine artery **130** is subdivided into smaller uterine vessels **170** (e.g., having a diameter of 2 millimeters or less) which feed fibroid **140.** The embolic particles **111** in the embolic composition partially or totally fill the lumen of uterine artery **130,** either partially or completely occluding the lumen of the uterine artery **130** that feeds uterine fibroid **140.**
In general, the particles are substantially formed of a polymer, such as a highly water insoluble, high molecular weight polymer. An example of such a polymer is a high molecular weight polyvinyl alcohol (PVA) that has been acetalized. The embolic particles can be substantially pure intrachain 1,3-acetalized PVA and substantially free of animal derived residue such as collagen. In embodiments, the particles include a minor amount (e.g., about 2.5 weight percent or less, about one weight percent or less, about 0.2 weight percent or less) of a gelling material (e.g., a polysaccharide, such as alginate).
**FIG. 2A** shows an embodiment in which the embolic particles have a substantially uniform spherical shape and size. **FIG. 2B** shows an embodiment in which an embolic particle has a well-defined outer spherical surface including relatively small, randomly located pores. The surface appears substantially smooth, with a surface morphology including larger features, such as crevice-like features. **FIGS. 2C-2E** show scanning electron micrograph (SEM) images of cross-sections through embolic particles in which the bodies of the particles define pores which provide compressibility and other properties to the embolic composition. Pores near the center of the particle are relatively large, and pores near the surface of the particle are relatively small.

The region of small pores near the surface of the embolic particle is relatively stiff and incompressible, which enhances resistance to shear forces and abrasion. In addition, the variable pore size profile can produce a symmetric compressibility and, it is believed, a compressibility profile. As a result, the particles can be relatively easily compressed from a maximum, at rest diameter to a smaller, compressed first diameter, although compression to an even smaller diameter requires substantially greater force. Without wishing to be bound by theory, it is believed that a variable compressibility profile can be due to the presence of a relatively weak, collapsible inter-pore wall structure in the center region where the pores are large, and a stiffer inter-pore wall structure near the surface of the particle, where the pores are more numerous and relatively small. It is further believed that a variable pore size profile can enhance elastic recovery after compression. It is also believed that the pore structure can influence the density of the embolic particles and the rate of carrier fluid or body fluid uptake.

In some embodiments, the embolic particles can be delivered through a catheter having a lumen with a cross-sectional area that is smaller (e.g., 50 percent or less) than the uncompressed cross-sectional area of the particles. In such embodiments, the embolic particles are compressed to pass through the catheter for delivery into the body. Typically, the compression force is provided indirectly, by depressing the syringe plunger to increase the pressure applied to the carrier fluid. In general, the embolic particles are relatively easily compressed to diameters sufficient for delivery through the catheter into the body. The relatively robust, rigid surface region can resist abrasion when the embolic particles contact hard surfaces such as syringe surfaces, hard plastic or metal stopcock surfaces, and the catheter lumen wall (made of, e.g., Teflon) during delivery. Once in the body, the embolic particles can substantially recover to original diameter and shape for efficient transport in the carrier and body fluid stream. At the point of occlusion, the particles can again compress as they aggregate in the occlusion region. The embolic particles can form a relatively dense occluding mass. The compression in the body is generally determined by the force provided by body fluid flow in the lumen. In some embodiments, the compression may be limited by the compression profile of the particles, and the number of embolic particles needed to occlude a given diameter may be reduced.

In some embodiments, among the particles delivered to a subject, the majority (e.g., 50 percent or more, 60 percent or more, 70 percent or more, 80 percent or more, 90 percent or more) of the particles have a diameter of 1500 microns or less (e.g., 1200 microns or less, 900 microns or less, 700 microns or less, 500 microns or less, 300 microns or less) and/or 10 microns or more (e.g., 100 microns or more, 300 microns or more, 400 microns or more, 500 microns or more, 700 microns or more, 900 microns or more).

In certain embodiments, the particles delivered to a subject have a mean diameter of 1500 microns or less (e.g., 1200 microns or less, 900 microns or less, 700 microns or less, 500 microns or less, 300 microns or less) and/or 10 microns or more (e.g., 100 microns or more, 300 microns or more, 400 microns or more, 500 microns or more, 700 microns or more, 900 microns or more). Exemplary ranges for the mean diameter of particles delivered to a subject include from 100 microns to 300 microns, from 300 microns to 500 microns, from 500 microns to 700 microns, and from 900 microns to 1200 microns. In general, a collection of particles has a mean diameter in approximately the middle of the range of the diameters of the individual particles, and a variance of 20 percent or less (e.g. 15 percent or less, 10 percent or less).

In some embodiments, the mean size of the particles delivered to a subject can vary depending upon the particular condition to be treated. As an example, in embodiments in which the particles are used to treat a liver tumor, the particles delivered to the subject can have a mean diameter of 500 microns or less (e.g., from 100 microns to 300 microns, from 300 microns to 500 microns). As another example, in embodiments in which the particles are used to treat a uterine fibroid, the particles delivered to the subject can have a mean diameter of 1200 microns or less (e.g., from 500 microns to 700 microns, from 700 microns to 900 microns, from 900 microns to 1200 microns).

As shown in FIG. 2C, in some embodiments a particle can be considered to include a center region, C, from the center c' of the particle to a radius of about r/3, a body region, B, from r/3 to 2 r/3 and a surface region, S, from 2r/3 to r. The regions can be characterized by the relative size of the pores in each region, the density of the pores (the number of pores per unit volume) in each region, and/or the material density (density of particle material per unit volume) in each region.

In general, the mean size of the pores in region C of a particle is greater than the mean size of the pores at region S of the particle. In some embodiments, the mean size of the pores in region C of a particle is greater than the mean size of the pores in region B the particle, and/or the mean size of the pores in region B of a particle is greater than the mean size of the pores at region S the particle. In some embodiments, the mean pore size in region C is about 20 microns or more (e.g., 30 microns or more, from 20 microns to 35 microns). In certain embodiments, the mean pore size in region B is 18 microns or less (e.g. 15 microns or less, from 18 microns to two microns). In some embodiments, the mean pore size of the pores in region S is one micron or less (e.g. from 0.1 micron to 0.01 micron). In certain embodiments, the mean pore size in region B is from 50 percent to 70 percent of the mean pore size in region C, and/or the mean pore size at region S is 10 percent or less (e.g., about two percent or less) of the mean pore size in region B. In some embodiments, the surface of a particle and/or its region S is/are substantially free of pores having a diameter greater than one micron (e.g., greater than 10 microns). In certain embodiments, the mean pore size in the region from

0.8r to r (e.g., from 0.9r to r) is one micron or less (e.g., 0.5 micron or less, 0.1 micron or less). In some embodiments, the region from the center of the particle to 0.9r (e.g., from the center of the particle to 0.8r) has pores of 10 microns or greater and/or has a mean pore size of from two microns to 35 microns. In certain embodiments, the mean pore size in the region from 0.8r to r (e.g., from 0.9r to r) is five percent or less (e.g., one percent or less, 0.3 percent or less) of the mean pore size in the region from the center to 0.9r. In some embodiments, the largest pores in the particles can have a size in the range of one percent or more (e.g., five percent or more, 10 percent or more) of the particle diameter. The size of the pores in a particle can be measured by viewing a cross-section as in **FIG. 2C.** For irregularly shaped (nonspherical) pores, the maximum visible cross-section is used. In **FIG. 2C,** the SEM was taken on wet particles including absorbed saline, which were frozen in liquid nitrogen and sectioned. **FIG. 2B** was taken prior to sectioning. In **FIGS. 2D-2E,** the particle was freeze-dried prior to sectioning and SEM analysis.

Generally, the density of pores in region C of a particle is greater than the density of pores at region S of the particle. In some embodiments, the density of pores in region C of a particle is greater than the density of pores in region B of the particle, and/or the density of pores in region B of a particle is greater than the density of pores at region S of the particle.

In general, the material density in region C of a particle is less than the material density at region S of the particle. In some embodiments, the material density in region C of a particle is less than the material density in region B of the particle, and/or the material density in region B of a particle is less than the material density at region S of the particle.

In general, the density of a particle (e.g., as measured in grams of material per unit volume) is such that it can be readily suspended in a carrier fluid (e.g., a pharmaceutically acceptable carrier, such as a saline solution, a contrast solution, or a mixture thereof) and remain suspended during delivery. In some embodiments, the density of a particle is from 1.1 grams per cubic centimeter to 1.4 grams per cubic centimeter. As an example, for suspension in a saline-contrast solution, the density can be from 1.2 grams per cubic centimeter to 1.3 grams per cubic centimeter.

In certain embodiments, the sphericity of a particle after compression in a catheter (e.g., after compression to 50 percent or more of the cross-sectional area of the particle) is 0.9 or more (e.g., 0.95 or more, 0.97 or more). A particle can be, for example, manually compressed, essentially flattened, while wet to 50 percent or less of its original diameter and then, upon exposure to fluid, regain a sphericity of 0.9 or more (e.g., 0.95 or more, 0.97 or more).

Manufacture

**FIG. 3A** shows an embodiment of a system for producing embolic particles. The system includes a flow controller **300,** a drop generator **310,** a gelling vessel **320,** a reactor vessel **330,** a gel dissolution chamber **340** and a filter **350.** As shown in **FIG. 3B,** flow controller **300** delivers polymer solutions to a viscosity controller **305,** which heats the solution to reduce viscosity prior to delivery to drop generator **310.** The solution passes through an orifice in a nozzle in drop generator **310,** forming drops of the solution. The drops are then directed into gelling vessel **320,** where the drops are stabilized by gel formation. The gel-stabilized drops are transferred from gelling vessel **320** to reactor vessel **330,** where the polymer in the gel-stabilized drops is reacted, forming precursor particles. The precursor particles are transferred to gel dissolution chamber **340,** where the gel is dissolved. The particles are then filtered in filter **350** to remove debris, and are sterilized and packaged as an embolic composition including embolic particles.

In general, a base polymer and a gelling precursor are dissolved in water and mixed.

Examples of base polymers include polyvinyl alcohols, polyacrylic acids, polymethacrylic acids, poly vinyl sulfonates, carboxymethyl celluloses, hydroxyethyl celluloses, substituted celluloses, polyacrylamides, polyethylene glycols, polyamides, polyureas, polyurethanes, polyesters, polyethers, polystyrenes, polysaccharides, polylactic acids, polyethylenes, polymethylmethacrylates, polycaprolactones, polyglycolic acids, poly(lactic-co-glycolic) acids (e.g., poly (d-lactic-co-glycolic) acids) and copolymers or mixtures thereof. A preferred polymer is polyvinyl alcohol (PVA). The polyvinyl alcohol, in particular, is typically hydrolyzed in the range of from about 80 percent to about 99 percent. The weight average molecular weight of the base polymer can be, for example, in the range of from 9000 to 186,000 (e.g., from 85,000 to 146,000, from 89,000 to 98,000).

Gelling precursors include, for example, alginates, alginate salts, xanthan gums, natural gum, agar, agarose, chitosan, carrageenan, fucoidan, furcellaran, laminaran, hypnea, eucheuma, gum arabic, gum ghatti, gum karaya, gum tragacanth, hyalauronic acid, locust beam gum, arabinogalactan, pectin, amylopectin, other water soluble polysaccharides and other ionically cross-linkable polymers. A particular gelling precursor is sodium alginate. A preferred sodium alginate is high guluronic acid, stem-derived alginate (e.g., about 50 percent or more, about 60 percent or more guluronic acid) with a low viscosity (e.g., from 20 mPas to 80 mPas at 20˚C), which produces a high tensile, robust gel.

In some embodiments, the base polymer (e.g., PVA, such as high molecular weight PVA) can be dissolved in water by heating (e.g., above 70˚C or more, about 121˚C), while the gelling precursor (e.g., an alginate) can be dissolved at room temperature. The base polymer (e.g., PVA) can be dissolved by mixing the base polymer and the gelling precursor (e.g., alginate) together in a vessel which is heated, e.g., to a temperature of at least about 50˚C (e.g., 65˚C

or more, 75˚C or more, 85˚C or more, 95˚C or more, 105˚C or more, 115˚C or more, about 121˚C). In some embodiments, the mixture can be heated in an autoclave. Alternatively, the base polymer (e.g., PVA) can be disposed in water and heated. The gelling precursor (e.g., alginate) can subsequently be added at room temperature, to avoid exposing the alginate to high temperature. Heat can also be applied, for example, by microwave application.

In certain embodiments, such as when the base polymer is PVA and the gelling precursor is alginate, the mixture can be from 6.5 weight percent to 8.5 weight percent (e.g., about eight weight percent, about seven weight percent) base polymer and from 1.5 weight percent to 2.5 weight percent (e.g., about 1.75 weight percent, about two weight percent) gelling precursor.

In some embodiments, the base polymer/gelling precursor mixture can be introduced to a high pressure pumping apparatus, such as a syringe pump (e.g., model PHD4400, Harvard Apparatus, Holliston, MA), and then transferred to drop generator **310.** Alternatively or additionally, drop generator **310** can contain a pressure control device that applies a pressure (e.g., from 0.5 Bar to 1.6 Bar) to the base polymer/gelling precursor mixture (a pressure head) to control the rate at which the mixture is transferred to drop generator **310.**

The pressure can be selected, for example, based on the size of the nozzle orifice and/or the desired viscosity of the base polymer/gelling precursor mixture, and/or the desired size of the particles. In general, for a given mixture, as the nozzle orifice is decreased, the pressure is increased. Generally, for a given mixture, as the desired viscosity of the mixture is decreased, the temperature is increased. As an example, in embodiments in which the nozzle orifice has a diameter of about 100 microns and the base polymer/gelling precursor mixture has a viscosity of from 60 mPas to 100 mPas, the pressure can be about 1.55 Bar. As another example, in embodiments in which the nozzle orifice has a diameter of about 200 microns and the base polymer/gelling precursor mixture has a viscosity of from 60 mPas to 100 mPas, the pressure can be about 0.55 Bar.

Referring to **FIG. 3B,** viscosity controller **305** is a heat exchanger that circulates water at a predetermined temperature about the flow tubing between the pump and drop generator 310. The base polymer/gelling precursor mixture flows into viscosity controller **305,** where the mixture is heated so that its viscosity is lowered to a desired level. Alternatively or additionally, the vessel containing the base polymer/gelling precursor mixture can be disposed in a heated fluid bath (e.g., a heated water bath) to heat the base polymer/gelling precursor mixture. In some embodiments (e.g., when the system does not contain viscosity controller **305**), flow controller **300** and/or drop generator **310** can be placed in a temperature-controlled chamber (e.g. an oven, a heat tape wrap) to heat the base polymer/gelling precursor mixture.

The temperature to which the base polymer/gelling precursor mixture is heated prior to transfer to drop generator **310** can be selected, for example, based on the desired viscosity of the mixture and/or the size of the orifice in the nozzle. In general, for a given mixture, the lower the desired viscosity of the mixture, the higher the temperature to which the mixture is heated. Generally, for a given mixture, the smaller the diameter of the nozzle, the higher the temperature to which the mixture is heated. As an example, in embodiments in which nozzle has a diameter of from 150 microns to 300 microns and the desired viscosity of the mixture is from 90 mPas to 200 mPas, the mixture can be heated to a temperature of from 60˚C to 70˚C (e.g., about 65˚C). As another example, in embodiments in which the nozzle has a diameter of from 100 microns to 200 microns and the desired viscosity of the mixture is from 60 mPas to 100 mPas, the mixture can be heated to a temperature of from 70˚C to 80˚C (e.g., about 75˚C).

Drop generator 310 generates substantially spherical drops of a predetermined diameter by forcing a stream of the base polymer/gelling precursor mixture through the nozzle orifice. The nozzle is subjected to a periodic disturbance to break up the jet stream of the mixture into drops of the mixture. The jet stream can be broken into drops by vibratory action generated, for example, by an electrostatic or piezoelectric element. The drop size can be controlled, for example, by controlling the nozzle orifice diameter, base polymer/gelling precursor flow rate, nozzle vibration amplitude, and nozzle vibration frequency. In general, holding other parameters constant, increasing the nozzle orifice diameter results in formation of larger drops, and increasing the flow rate results in larger drops. Generally, holding other parameters constant, increasing the nozzle vibration amplitude results in larger drops, and reducing the nozzle vibration frequency results in larger drops. In general, the nozzle orifice diameter can be 500 microns or less (e.g., 400 microns or less, 300 microns or less, 200 microns or less, 100 microns or less) and/or 50 microns or more. The flow rate through the drop generator is typically from about one milliliter per minute to about 12 milliliters per minute. Generally, the nozzle frequency used can be 0.1 KHz or more (e.g., 0.8 KHz or more, 1.5 KHz or more, 1.75 KHz or more, 1.85 KHz or more, 2.5 KHz or more, from 0.1 KHz to 0.8 KHz). In general, the nozzle vibration amplitude is larger than the width of the jet stream. The drop generator can have a variable nozzle vibration amplitude setting, such that an operator can adjust the amplitude of the nozzle vibration. In some embodiments, the nozzle vibration amplitude is set at between about 80 percent and about 100 percent of the maximum setting.

In some embodiments, drop generator **310** can charge the drops after formation, such that mutual repulsion between drops prevents drop aggregation as the drops travel from drop generator **310** to gelling vessel **320.** Charging may be achieved, for example, by an electrostatic charging device such as a charged ring positioned downstream of the nozzle.

An example of a commercially available electrostatic drop generator is the model NISCO Encapsulation unit VAR D (NISCO Engineering, Zurich, Switzerland). Another example of a commercially available drop generator is the Inotech Encapsulator unit IE-50R/NS (Inotech AG, Dottikon, Switzerland).

Drops of the base polymer and gelling precursor mixture are captured in gelling vessel **320.** The distance between gelling vessel 320 and the orifice of the nozzle in drop generator **310** is generally selected so that the jet stream of the base polymer/gelling precursor mixture is substantially broken up into discrete drops before reaching gelling vessel **320.** In some embodiments, the distance from the nozzle orifice to the mixture contained in gelling vessel **320** is from 12,7 cm (five inches) to 15.24 cm (six inches).

The mixture contained in gelling vessel 320 includes a gelling agent which interacts with the gelling precursor to stabilize drops by forming a stable gel. Suitable gelling agents include, for example, a divalent cation such as alkali metal salt, alkaline earth metal salt or a transition metal salt that can ionically cross-link with the gelling agent. An inorganic salt, for example, a calcium, barium, zinc or magnesium salt can be used as a gelling agent. In embodiments, particularly those using an alginate gelling precursor, a suitable gelling agent is calcium chloride. The calcium cations have an affinity for carboxylic groups in the gelling precursor. The cations complex with carboxylic groups in the gelling precursor, resulting in encapsulation of the base polymer in a matrix of gelling precursor.

Without wishing to be bound by theory, it is believed that in some embodiments (e.g., when forming particles having a diameter of 500 microns or less), it can be desirable to reduce the surface tension of the mixture contained in gelling vessel **320.** This can be achieved, for example, by heating the mixture in gelling vessel **320** (e.g., to a temperature greater than room temperature, such as a temperature of 30°C or more), by bubbling a gas (e.g., air, nitrogen, argon, krypton, helium, neon) through the mixture contained in gelling vessel **320,** by stirring (e.g., via a magnetic stirrer) the mixture contained in gelling vessel **320,** by including a surfactant in the mixture containing the gelling agent, and/or by forming a mist containing the gelling agent above the mixture contained in gelling vessel **320** (e.g., to reduce the formation of tails and/or enhance the sphericity of the particles).

**FIG. 4** shows a photo-image of the gelled particles. As evident, a pore structure in the particle forms in the gelling stage. The concentration of the gelling agent can affect pore formation in the particle, thereby controlling the porosity gradient in the particle. Adding non-gelling ions (e.g., sodium ions) to the gelling solution can reduce the porosity gradient, resulting in a more uniform intermediate porosity throughout the particle. In embodiments, the gelling agent is, for example, from 0.01 weight percent to 10 weight percent (e.g., from one weight percent to five weight percent, about two weight percent) in deionized water. In embodiments, particles, including gelling agent and a pore structure, can be used in embolic compositions.

Following drop stabilization, the gelling solution can be decanted from the solid drops, or the solid drops can be removed from the gelling solution by sieving. The solid drops are then transferred to reactor vessel 330, where the base polymer in the solid drops is reacted (e.g., cross-linked) to produce precursor particles.

Reactor vessel 330 contains an agent that chemically reacts with the base polymer to cause cross-linking between polymer chains and/or within a polymer chain. The agent diffuses into the solid drops from the surface of the particle in a gradient which, it is believed, provides more cross-linking near the surface of the solid drop than in the body and center of the drop. Reaction is greatest at the surface of a solid drop, providing a stiff, abrasion-resistant exterior. For polyvinyl alcohol, for example, vessel 330 includes one or more aldehydes, such as formaldehyde, glyoxal, benzaldehyde, aterephthalaldehyde, succinaldehyde and glutaraldehyde for the acetalization of polyvinyl alcohol. Vessel 330 also includes an acid, for example, strong acids such as sulfuric acid, hydrochloric acid, nitric acid and weak acids such as acetic acid, formic acid and phosphoric acid. In embodiments, the reaction is primarily a 1,3-acetalization:

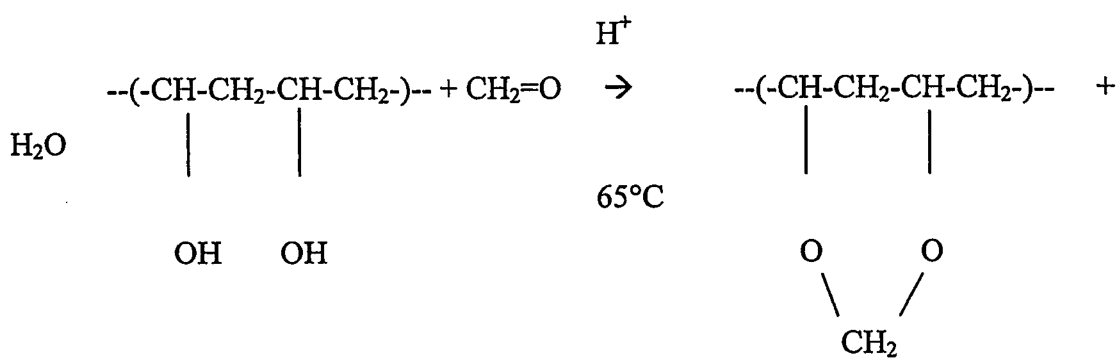


This intra-chain acetalization reaction can be carried out with relatively low probability of inter-chain cross-linking, as described in John G. Pritchard, "Poly(Vinyl Alcohol) Basic Properties and Uses (Polymer Monograph, vol. 4)

(see p. 93-97), Gordon and Breach, Science Publishers Ltd., London, 1970, which is incorporated herein by reference. Because the reaction proceeds in a random fashion, some OH groups along a polymer chain might not react with adjacent groups and may remain unconverted.

Adjusting for the amounts of aldehyde and acid used, reaction time and reaction temperature can control the degree of acetalization. In embodiments, the reaction time is from five minutes to one hour (e.g., from 10 minutes to 40 minutes, about 20 minutes). The reaction temperature can be, for example, from 25°C to 150°C (e.g., from 75°C to 130°C, about 65°C). Reactor vessel **330** can be placed in a water bath fitted with an orbital motion mixer. The cross-linked precursor particles are washed several times with deionized water to neutralize the particles and remove any residual acidic solution.

The precursor particles are transferred to dissolution chamber **340,** where the gelling precursor is removed (e.g., by an ion exchange reaction). In embodiments, sodium alginate is removed by ion exchange with a solution of sodium hexa-metaphosphate (EM Science). The solution can include, for example, ethylenediaminetetracetic acid (EDTA), citric acid, other acids, and phosphates. The concentration of the sodium hexa-metaphosphate can be, for example, from one weight percent to 20 weight percent (e.g., from one weight percent to ten weight percent, about five weight percent) in deionized water. Residual gelling precursor (e.g., sodium alginate) can be measured by assay (e.g., for the detection of uronic acids in, for example, alginates containing mannuronic and guluronic acid residues). A suitable assay includes rinsing the particles with sodium tetraborate in sulfuric acid solution to extract alginate, combining the extract with metahydroxydiphenyl colormetric reagent, and determining concentration by UV/VIS spectroscopy. Testing can be carried out by alginate suppliers such as FMC Biopolymer, Oslo, Norway. Residual alginate may be present in the range of, for example, from about 20 weight percent to about 35 weight percent prior to rinsing, and in the range of from 0.01 weight percent to 0.5 weight percent (e.g., from 0.1 weight percent to 0.3 weight percent, about 0.18 weight percent) in the particles after rinsing for 30 minutes in water at about 23°C.

The particles are filtered through filter 350 to remove residual debris. Particles of from 100 microns to 300 microns can filtered through a sieve of about 710 microns and then a sieve of about 300 microns. The particles can then be collected on a sieve of about 20 microns. Particles of from 300 to 500 microns can filtered through a sieve of about 710 microns and then a sieve of about 500 microns. The particles can then be collected on a sieve of about 100 microns. Particles of from 500 to 700 microns can be filtered through a sieve of about 1000 microns, then filtered through a sieve of about 710 microns, and then a sieve of about 300 microns. The particles can then be collected in a catch pan. Particles of from 700 to 900 microns can be filtered through a sieve of 1000 microns and then a sieve of 500 microns. The particles can then be collected in a catch pan. Particles of from 900 to 1200 microns can filtered through a sieve of 1180 microns and then a sieve of 710 microns. The particles can then be collected in a catch pan.

The particles are then packaged. Typically, from one milliliter to five milliliters of particles are packaged in from five milliliters to ten milliliters of saline. The filtered particles then are typically sterilized by a low temperature technique, such as e-beam irradiation. In embodiments, electron beam irradiation can be used to pharmaceutically sterilize the particles (e.g., to reduce bioburden). In e-beam sterilization, an electron beam is accelerated using magnetic and electric fields, and focused into a beam of energy. The resultant energy beam can be scanned by means of an electromagnet to produce a "curtain" of accelerated electrons. The accelerated electron beam penetrates the collection of particles, destroying bacteria and mold to sterilize and reduce the bioburden in the particles. Electron beam sterilization can be carried out by sterilization vendors such as Titan Scan, Lima, Ohio.

The embolic compositions can be used in the treatment of, for example, fibroids, tumors, internal bleeding, AVMs, hypervascular tumors, fillers for aneurysm sacs, endoleak sealants, arterial sealants, puncture sealants and occlusion of other lumens such as fallopian tubes. Fibroids can include uterine fibroids which grow within the uterine wall (intramural type), on the outside of the uterus (subserosal type), inside the uterine cavity (submucosal type), between the layers of broad ligament supporting the uterus (interligamentous type), attached to another organ (parasitic type), or on a mushroom-like stalk (pedunculated type). Internal bleeding includes gastrointestinal, urinary, renal and varicose bleeding. AVMs are for example, abnormal collections of blood vessels, e.g. in the brain, which shunt blood from a high pressure artery to a low pressure vein, resulting in hypoxia and malnutrition of those regions from which the blood is diverted.

The magnitude of a dose of an embolic composition can vary based on the nature, location and severity of the condition to be treated, as well as the route of administration. A physician treating the condition, disease or disorder can determine an effective amount of embolic composition. An effective amount of embolic composition refers to the amount sufficient to result in amelioration of symptoms or a prolongation of survival of the patient. The embolic compositions can be administered as pharmaceutically acceptable compositions to a patient in any therapeutically acceptable dosage, including those administered to a patient intravenously, subcutaneously, percutaneously, intratrachealy, intramuscularly, intramucosaly, intracutaneously, intra-articularly, orally or parenterally.

In some embodiments, a composition containing the particles can be used to prophylactically treat a condition.

Compositions containing the particles can be prepared in calibrated concentrations of the particles for ease of delivery by the physician. Suspensions of the particles in saline solution can be prepared to remain stable (e.g., to not

precipitate) over a duration of time. A suspension of the particles can be stable, for example, for from one minute to 20 minutes (e.g. from one minute to ten minutes, from two minutes to seven minutes, from three minutes to six minutes). The concentration of particles can be determined by adjusting the weight ratio of the particles to the physiological solution. If the weight ratio of the particles is too small, then too much liquid could be injected into a blood vessel, possibly allowing the particles to stray into lateral vessels. In some embodiments, the physiological solution can contain from 0.01 weight percent to 15 weight percent of the particles. A composition can include a mixture of particles, such as particles having the pore profiles discussed above, particles with other pore profiles, and/or non-porous particles.

While certain embodiments have been described, the invention is not so limited.

As an example, particles can be used for embolic applications without removal of the gelling agent (e.g. alginate). Such particles can be prepared, for example, as described above, but without removing the alginate from the particle after cross-linking.

As another example, while substantially spherical particles are preferred, nonspherical particles can be manufactured and formed by controlling, for example, drop formation conditions. In some embodiments, nonspherical particles can be formed by post-processing the particles (e.g., by cutting or dicing into other shapes).

Moreover, in some embodiments the particles can include one or more therapeutic agents (e.g., drugs). The therapeutic agent(s) can be in and/or on the particles. Therapeutic agents include agents that are negatively charged, positively charged, amphoteric, or neutral. Therapeutic agents can be, for example, materials that are biologically active to treat physiological conditions; pharmaceutically active compounds; gene therapies; nucleic acids with and without carrier vectors; oligonucleotides; gene/vector systems; DNA chimeras; compacting agents (e.g., DNA compacting agents); viruses; polymers; hyaluronic acid; proteins (e.g., enzymes such as ribozymes); cells (of human origin, from an animal source, or genetically engineered); stem cells; immunologic species; nonsteroidal anti-inflammatory medications; oral contraceptives; progestins; gonadotrophin-releasing hormone agonists; chemotherapeutic agents; and radioactive species (e.g., radioisotopes, radioactive molecules). Non-limiting examples of therapeutic agents include anti-thrombogenic agents; antioxidants; angiogenic and anti-angiogenic agents and factors; anti-proliferative agents (e.g., agents capable of blocking smooth muscle cell proliferation); anti-inflammatory agents; calcium entry blockers; antineoplastic/antiproliferative/anti-mitotic agents (e.g., paclitaxel, doxorubicin, cisplatin); antimicrobials; anesthetic agents; anti-coagulants; vascular cell growth promoters; vascular cell growth inhibitors; cholesterol-lowering agents; vasodilating agents; agents which interfere with endogenous vasoactive mechanisms; and survival genes which protect against cell death. Therapeutic agents are described in US 2004096662 entitled "Agent Delivery Particle".

In addition, in some embodiments (e.g., where the base polymer is a polyvinyl alcohol and the gelling precursor is alginate), after contacting the particles with the gelling agent but before cross-linking, the particles can be physically deformed into a specific shape and/or size. For example, the particles can be molded, compressed, punched, and/or agglomerated with other particles. After shaping, the base polymer (e.g., polyvinyl alcohol) can be cross-linked, optionally followed by substantial removal of the gelling precursor (e.g., alginate). Particle shaping is described, for example, in US 2003203985 entitled "Forming a Chemically Cross-Linked Particle of a Desired Shape and Diameter".

Furthermore, in some embodiments the particles can be used for tissue bulking. As an example, the particles can be placed (e.g., injected) into tissue adjacent a body passageway. The particles can narrow the passageway, thereby providing bulk and allowing the tissue to constrict the passageway more easily. The particles can be placed in the tissue according to a number of different methods, for example, percutaneously, laparoscopically, and/or through a catheter. In certain embodiments, a cavity can be formed in the tissue, and the particles can be placed in the cavity. Particle tissue bulking can be used to treat, for example, intrinsic sphincteric deficiency (ISD), vesicoureteral reflux, gastroesophageal reflux disease (GERD), and/or vocal cord paralysis (e.g., to restore glottic competence in cases of paralytic dysphonia). In some embodiments, particle tissue bulking can be used to treat urinary incontinence and/or fecal incontinence. The particles can be used as a graft material or a filler to fill and/or to smooth out soft tissue defects, such as for reconstructive or cosmetic applications (e.g., surgery). Examples of soft tissue defect applications include cleft lips, scars (e.g., depressed scars from chicken pox or acne scars), indentations resulting from liposuction, wrinkles (e.g., glabella frown wrinkles), and soft tissue augmentation of thin lips. Tissue bulking is described, for example, in US 2003233150 entitled "Tissue Treatment".

The following examples are intended as illustrative and nonlimiting.

## Example 1

Particles were prepared as follows.

An aqueous solution containing eight weight percent polyvinyl alcohol (99+ percent hydrolyzed, average $M_w$ 89,000-120,000 (Aldrich)) and two weight percent sodium alginate (PRONOVA UPLVG, (FMC BioPolymer, Princeton, NJ)) in deionized water was prepared. The solution was heated to about 121˚C. The solution had a viscosity of about 310 centipoise at room temperature and a viscosity of about 160 centipoise at 65˚C. Using a model PHD4400 syringe pump (Harvard Apparatus, Holliston, MA), the mixture was fed into a model NISCO Encapsulation unit VAR D drop

generator (NISCO Engineering, Zurich, Switzerland). Drops generated by the drop generator were directed into a gelling vessel containing two weight percent calcium chloride in deionized water, and stirred with a stirring bar. The calcium chloride solution was decanted within about three minutes to avoid substantial leaching of the polyvinyl alcohol from the drops into the solution. The drops were added to a reaction vessel containing a solution of four weight percent formaldehyde (37 weight percent in methanol) and 20 weight percent sulfuric acid (95-98 percent concentrated). The reaction solution was stirred at 65˚C for 20 minutes. Precursor particles were rinsed with deionized water (3 x 300 milliliters) to remove residual acidic solution. The sodium alginate was substantially removed by soaking the precursor particles in a solution of five weight percent sodium hexa-methaphosphate in deionized water for 0.5 hour. The solution was rinsed in deionized water to remove residual phosphate and alginate. The particles were filtered by sieving, as discussed above, placed in saline (USP 0.9 percent NaCl) and sterilized by irradiation sterilization.

Particles were produced at the nozzle diameters, nozzle frequencies and flow rates (amplitude about 80 percent of maximum) described in Table I.

TABLE I

| Particle Size (microns) | Nozzle Diameter (microns) | Frequency (kHz) | Flow Rate (mL/min) | Density (g/mL) | Sphericity | Suspendability (minutes) |
|---|---|---|---|---|---|---|
| 500-700 | 150 | 0.45 | 4 | - | 0.92 | 3 |
| 700-900 | 200 | 0.21 | 5 | 1.265 | 0.94 | 5 |
| 900-1200 | 300 | 0.22 | 10 | - | 0.95 | 6 |

Suspendability was measured at room temperature by mixing a solution of two milliliters of particles in five milliliters of saline with contrast solution (Omnipaque 300, Nycomed, Buckinghamshire, UK), and observing the time for about 50 percent of the particles to enter suspension (i.e., not to have sunk to the bottom or floated to the top of a container having a volume of about ten milliliters and a diameter of about 25 millimeters). Suspendability provides a practical measure of how long the particles will remain suspended in use.

Measurements were also made of the amount of time that the particles remained suspended in the contrast solution. The particles remained in suspension for from about two to about three minutes.

Omnipaque 300 is an aqueous solution of Iohexol, N.N.-Bis (2,3-dihydroxypropyl)-T-[N-(2,3-dihydroxypropyl)-acetamide]-2,4,6-trilodo-isophthalamide. Omnipaque 300 contains 647 milligrams of iohexol equivalent to 300 milligrams of organic iodine per milliliter. The specific gravity of Omnipaque 300 is 1.349 of 37˚C, and Omnipaque 300 has an absolute viscosity 11.8 mPas at 20˚C.

Particle size uniformity and sphericity were measured using a Beckman Coulter RapidVUE Image Analyzer version 2.06 (Beckman Coulter, Miami, FL). Briefly, the RapidVUE takes an image of continuous-tone (gray-scale) form and converts it to a digital form through the process of sampling and quantization. The system software identifies and measures particles in an image in the form of a fiber, rod or sphere. Sphericity computation and other statistical definitions are in Appendix A, attached, which is a page from the RapidVUE operating manual.

Referring to **FIG. 5,** particle size uniformity is illustrated for particles having a diameter of from 700 microns to 900 microns. The x-axis is the particle diameter, and the y-axis is the volume-normalized percentage of particles at each particle size. The total volume of particles detected was computed, and the volume of the particles at each diameter was divided by the total volume. The embolic particles had a distribution of particle sizes with variance of less than ± 15 percent.

Example 2

Particles were prepared as follows.

An aqueous solution containing 7.06 weight percent polyvinyl alcohol (99+ percent hydrolyzed, average $M_w$ 89,000-120,000 (Aldrich)) and 1.76 weight percent sodium alginate (PRONOVA UPLVG, (FMC BioPolymer, Princeton, NJ)) was prepared. The solution was heated to about 121 ˚C. The solution had a viscosity of about 140 mPas at room temperature, and a viscosity of about 70 mPas at 65˚C. Using a pressurized vessel, the mixture was fed to a drop generator (Inotech Encapsulator unit IE-50R/NS, Inotech Biosystems International, Inc.). Drops generated by the drop generator were directed into a gelling vessel containing two weight percent calcium chloride in deionized water, and stirred with a stirring bar. The drops were collected within about three minutes to avoid substantial leaching of the polyvinyl alcohol from the drops into the solution. The drops were added to a reaction vessel containing a solution of four weight percent formaldehyde (37 weight percent in methanol) and 20 weight percent sulfuric acid (95-98 percent

concentrated). The reaction solution was stirred at 65°C for 20 minutes. The precursor particles were rinsed with deionized water (3 x 300 milliliters) to remove residual acidic solution. The sodium alginate was substantially removed by soaking the precursor particles in a solution of five weight percent sodium hexa-methaphosphate in deionized water for half an hour. The solution was rinsed in deionized water to remove residual phosphate and alginate. The particles were filtered by sieving, placed in saline (USP 0.9 percent NaCl) and sterilized by irradiation sterilization.

The particles were produced at the nozzle diameters, nozzle frequencies and pressures (amplitude about 80 percent of maximum) described in Table II.

TABLE II

| Particle Size (microns) | Nozzle Diameter (microns) | Frequency (KHz) | Pressure (Bar) | Flow Rate (mL/min) | Suspendability (minutes) |
|---|---|---|---|---|---|
| 100-300 | 100 | 2.5 | 1.55 | 2.5 | 0.25 |
| 300-500 | 200 | 1.85 | 0.55 | 6.8 | 1 |

Suspendability was measured as described in Example 1.

Measurements were also made of the amount of time that the particles remained suspended in the contrast solution. The particles remained suspended in the contrast solution for about 20 minutes.

**FIG. 6** shows particle size uniformity for particles having a diameter of from 300 microns to 500 microns (see discussion in Example 1). The embolic particles had a distribution of particle sizes with a variance of less than about ± 15 percent.

<u>Example 3</u>

Referring to **FIG. 7,** a catheter compression test was used to investigate the injectability, and indirectly, the compressibility, of the particles. The test apparatus included a reservoir syringe **610** and an injection syringe **620** coupled to a T-valve **630.** Reservoir syringe **610** was a 20 milliliter syringe while injection syringe **620** was a three milliliter syringe. T-valve **630** was coupled in series to a second T-valve **640.** T-valve **640** was coupled to a catheter **650** and a pressure transducer **660.** Injection syringe **620** was coupled to a syringe pump **621** (Harvard Apparatus).

To test deliverability of the particles, syringes **610** and **620** were loaded with embolic composition in saline and contrast agent (50/50 Ominipaque 300). The embolic composition in syringes **610** and **620** was intermixed by turning the T-valve to allow fluid between the syringes to mix and suspend the particles. After mixing, the embolic composition in syringe **620** flowed at a rate of about ten milliliters per minute. The back pressure generated in catheter **650** was measured by the pressure transducer **660** in millivolts to measure the clogging of catheter **650.** About one milliliter of the particles was mixed in ten milliliters of solution.

Results for several different catheters (available from Boston Scientific, Natick, MA) and particle sizes are shown in Table III. The baseline pressure was the pressure observed when injecting carrier fluid only. The delivery pressure was the pressure observed while delivering particles in carrier fluid. The average was the average of the peak pressure observed in the three runs.

TABLE III

| SIZE (microns) | Delivery Catheter | Inner Diameter (microns) | Avg. Baseline Pressure (psia) | Avg. Delivery Pressure (psia) | Total number of Clogs |
|---|---|---|---|---|---|
| 100-300 | Spinnaker Elite® | 279 | 71.3 | 65.4 | 0 |
| 300-500 | Spinnaker Elite® | 330 | 54.6 | 52.6 | 0 |
| 500-700 | RENEGADE® | 533 | 32.610 | 33.245 | 0 |
| 700-900 | FASTRACKER® | 609 | 11.869 | 13.735 | 0 |
| 900-1200 | GLIDECATH® | 965 | 0.788 | 0.864 | 0 |

As evident, particles in each of the size ranges were successfully delivered without clogging catheters with a lumen diameter smaller than the largest particle size. The particles exhibited a post-compression sphericity of 0.9 or more.

Example 4

Solubility was tested by mixing particles in a solution of solvent at room temperature for about 0.5 hour and observing the mixture for visible signs of dissolution. The particles were insoluble in DMSO (dimethylsulfoxide), HFIP (hexafluoroisopropanol), and THF (tetrahydrofuran).

Example 5

Particles had the following glass transition temperatures, as measured by differential scanning calorimetry data (DSC):

| Size (microns) | Glass Transition Temperature (˚C) |
|---|---|
| 100-300 | 107-108 |
| 300-500 | 110-111 |
| 500-700 | 109.30-110.14 |
| 900-1200 | 108.30-111.87 |

Example 6

**FIGS. 8 and 9** show the ATR infrared spectra of dried particles prepared according to Examples 1 and 2, respectively.

Other embodiments are in the claims.

Appendix A

5. DEFINITIONS OF VARIABLES

5.1 STATISTICAL MEASURES

Averages:

| | | |
|---|---|---|
| Arithmetic mean: | $\sum_k D_k / N$ | Average diameter of all the particles in the sample. |
| Surface mean: | $[\sum_k D_k^2 / N]^{1/2}$ | The diameter of a particle whose surface area, it multiplied by the total number of particles, will equal the total surface area of the sample. |
| Volume mean: | $[\sum_k D_k^3 / N]^{1/3}$ | The diameter of a particle whose volume, it multiplied by the total number of particles, equals the total volume of the sample. |
| Sauter mean: | $\sum_k D_k^3 / \sum_k D_k^2$ | The diameter of a particle whose ratio of volume to surface area is the same as the complete sample. |
| Sample size check: | $\sum Dmax^3 / \sum_k D_k^3$ | Fraction of the total volume represented by the largest particle. |

Percentiles:

DVXX : the XXth percentile by volume. It is computed as the diameter such that the collection of particles having that size or less represents XX % of the total volume. The commonly used ones are DV10, DV50 and DV90. DV50 is also called the volume median. It is the diameter that divides the sample into two equal halves, by mass or volume.

Measures of spread:

Deviation: $$\frac{\Sigma\{[D_{v0.5} - (D_{klb} + D_{kub})] / 2\}N_k}{\sum_k N_k D_{v0.5}}$$

klb = lower bound of bin k
kub = upper bound of bin k

Relative span: $$\frac{D_{v0.9} - D_{v0.1}}{D_{v0.5}}$$

Sphericity: a value from 0 to 1, with 1 representing a perfect circle. Computed as Da / $Dp_r$ where Da = square root ( 4 A / π), Dp = P /π; A = pixel area, P = pixel perimeter.

## Claims

**1.** A polymeric particle suitable to be part of a composition for embolization having a diameter of 500 microns or less, wherein the particle has a first density of pores in an interior region and a second density of pores at a surface region, the first density being different from the second density.

**2.** The polymeric particle of claim 1, wherein the first density is greater than the second density.

**3.** The polymeric particle of claim 1, wherein the particle has a first average pore size in the interior region and a second average pore size at the surface region, the first average pore size being different from the second average pore size.

**4.** The polymeric particle of claim 3, wherein the first average pore size is greater than the second average pore size.

**5.** The polymeric particle of claim 1, wherein the particle has a diameter of 10 microns or more.

**6.** The polymeric particle of claim 1, wherein the particle has a diameter of 100 microns or more.

**7.** The polymeric particle of claim 6, wherein the particle has a diameter of 300 microns or less.

**8.** The polymeric particle of claim 1, wherein the particle has a diameter of 300 microns or more.

**9.** The polymeric particle of claim 1, wherein the particle comprises at least one polymer selected from the group consisting of polyvinyl alcohols, polyacrylic acids, polymethacrylic acids, poly vinyl sulfonates, carboxymethyl celluloses, hydroxyethyl celluloses, substituted celluloses, polyacrylamides, polyethylene glycols, polyamides, polyureas, polyurethanes, polyesters, polyethers, polystyrenes, polysaccharides, polylactic acids, polyethylenes, polymethylmethacrylates, polycaprolactones; polyglycolic acids, and poly(lactic-co-glycolic) acids.

**10.** The polymeric particle of claim 1, wherein the particle comprises a polyvinyl alcohol.

**11.** The polymeric particle of claim 1, wherein the particle is at least partially coated with a bioabsorbable material.

**12.** The polymeric particle of claim 1, wherein the particle has a density of from 1.1 grams per cubic centimeter to 1.4 grams per cubic centimeter.

**13.** The polymeric particle of claim 1, wherein the particle has a sphericity of 0.9 or more.

**14.** The polymeric particle of claim 1, wherein, after compression to about 50 percent, the particle has a sphericity of 0.9 or more.

**15.** The polymeric particle of claim 1, wherein the particle comprises 2.5 weight percent or less polysaccharide.

**16.** The polymeric particle of claim 15, wherein the polysaccharide comprises alginate.

**17.** The polymeric particle of claim 16, wherein the alginate has a guluronic acid content of 60 percent or greater.

**18.** The polymeric particle of claim 1, wherein the particle is insoluble in DMSO.

**19.** The polymeric particle of claim 1, wherein the particle is substantially free of animal-derived compounds.

**20.** A polymeric particle suitable to be part of a composition for embolization having a diameter of 500 microns or less, wherein the particle has a first average pore size in an interior region and a second average pore size at a surface region, the first average pore size being different from the second average pore size.

**21.** The polymeric particle of claim 20, wherein the first average pore size is greater than the second average pore size.

**22.** A composition suitable for embolization, comprising:

a plurality of polymeric particles, at least some of the plurality of particles having a diameter of 500 microns or less, wherein at least some of the particles having a diameter of 500 microns or less have a first density of pores in an interior region and a second density of pores at a surface region, the first density being different from the second density; and
a carrier fluid, the plurality of particles being in the carrier fluid.

**23.** The composition of claim 22, wherein the carrier fluid comprises a saline solution.

**24.** The composition of claim 22, wherein the carrier fluid comprises a contrast agent.

**25.** The composition of claim 22, wherein the plurality of particles has a mean diameter of 500 microns or less.

**26.** The composition of claim 22, wherein the plurality of particles has a mean diameter of 10 microns or more.

**27.** The composition of claim 22, wherein the plurality of particles has a mean diameter of 100 microns or more.

**28.** The composition of claim 27, wherein the plurality of particles has a mean diameter of 300 microns or less.

**29.** The composition of claim 22, wherein the plurality of particle has a mean diameter of 300 microns or more.

**30.** A composition suitable for embolization comprising:

a plurality of polymeric particles, at least some of the plurality of particles having a diameter of 500 microns or less; wherein at least some of the particles having a diameter of 500 microns or less have a first average pore size in an interior region and a second average pore size at a surface region, the first average pore size being different from the second average pore size; and
a carrier fluid, the plurality of particles being in the carrier fluid.

**31.** A method for preparing polymeric particles as defined in one of claims 1 to 19, comprising:

forming drops containing a base polymer and a gelling precursor; and
contacting the drops with a gelling agent to form particles containing the base polymer and the gelling precursor, the gelling agent being contained in a vessel, and the method further including at least one member selected from the group consisting of bubbling a gas through the gelling agent, including a surfactant in the mixture containing the gelling agent, disposing a mist containing the gelling agent between a source of the drops and the vessel, and stirring the gelling agent.

**Patentansprüche**

**1.** Polymeres Teilchen, geeignet, Teil einer Zusammensetzung für Embolisation zu sein, mit einem Durchmesser von 500 Micron oder weniger, wobei das Teilchen eine erste Porendichte in einem inneren Bereich und eine zweite Porendichte in einem Oberflächenbereich hat, wobei die erste Dichte verschieden von der zweiten Dichte ist.

2. Polymeres Teilchen nach Anspruch 1, wobei die erste Dichte größer als die zweite Dichte ist.

3. Polymeres Teilchen nach Anspruch 1, wobei das Teilchen eine erste durchschnittliche Porengröße in dem inneren Bereich und eine zweite durchschnittliche Porengröße in dem Oberflächenbereich hat, wobei die erste durchschnittliche Porengröße verschieden von der zweiten durchschnittlichen Porengröße ist.

4. Polymeres Teilchen nach Anspruch 3, wobei die erste durchschnittliche Porengröße größer als die zweite durchschnittliche Porengröße ist.

5. Polymeres Teilchen nach Anspruch 1, wobei das Teilchen einen Durchmesser von 10 Micron oder mehr hat.

6. Polymeres Teilchen nach Anspruch 1, wobei das Teilchen einen Durchmesser von 100 Micron oder mehr hat.

7. Polymeres Teilchen nach Anspruch 6, wobei das Teilchen einen Durchmesser von 300 Micron oder weniger hat.

8. Polymeres Teilchen nach Anspruch 1, wobei das Teilchen einen Durchmesser von 300 Micron oder mehr aufweist.

9. Polymeres Teilchen nach Anspruch 1, wobei das Teilchen mindestens ein Polymer umfasst, ausgewählt aus der Gruppe bestehend aus Polyvinylalkoholen, Polyacrylsäuren, Polymethacrylsäuren, Polyvinylsulfonaten, Carboxylmethylzellulosen, Hydroxyethylzellulosen, substituierten Zellulosen, Polyacrylamiden, Polyethylenglykolen, Polyamiden, Polyharnstoffen, Polyurethanen, Polyestern, Polyethem, Polystyrolen, Polysacchariden, Polymilchsäuren, Polyethylenen, Polymethylmethacrylaten, Polycaprolactonen, Polyglykolsäuren und Milchsäure-Glykolsäure-Copolymeren.

10. Polymeres Teilchen nach Anspruch 1, wobei das Teilchen einen Polyvinylalkohol umfasst.

11. Polymeres Teilchen nach Anspruch 1, wobei das Teilchen mindestens teilweise mit einem bioabsorbierbaren Material beschichtet ist.

12. Polymeres Teilchen nach Anspruch 1, wobei das Teilchen eine Dichte von 1,1 Gramm pro Kubikzentimeter bis 1,4 Gramm pro Kubikzentimeter hat.

13. Polymeres Teilchen nach Anspruch 1, wobei das Teilchen eine Kugelförmigkeit von 0,9 oder mehr hat.

14. Polymeres Teilchen nach Anspruch 1, wobei nach Kompression auf ungefähr 50% das Teilchen eine Kugelförmigkeit von 0,9 oder mehr hat.

15. Polymeres Teilchen nach Anspruch 1, wobei das Teilchen 2,5 Gew.-% oder weniger Polysaccharid umfasst.

16. Polymeres Teilchen nach Anspruch 15, wobei das Polysaccharid Alginat umfasst.

17. Polymeres Teilchen nach Anspruch 16, wobei das Alginat einen Guluronsäuregehalt von 60 Prozent oder mehr hat.

18. Polymeres Teilchen nach Anspruch 1, wobei das Teilchen in DMSO unlöslich ist.

19. Polymeres Teilchen nach Anspruch 1, wobei das Teilchen im Wesentlichen frei von tierabgeleiteten Verbindungen ist.

20. Polymeres Teilchen, geeignet, Teil einer Zusammensetzung für Embolisation zu sein, mit einem Durchmesser von 500 Micron oder weniger, wobei das Teilchen eine erste durchschnittliche Porengröße in einem inneren Bereich und eine zweite durchschnittliche Porengröße in einem Oberflächenbereich hat, wobei die erste durchschnittliche Porengröße verschieden von der zweiten durchschnittlichen Porengröße ist.

21. Polymeres Teilchen nach Anspruch 20, wobei die erste durchschnittliche Porengröße größer als die zweite durchschnittliche Porengröße ist.

22. Zusammensetzung, geeignet für Embolisation, aufweisend:

eine Vielzahl von polymeren Teilchen, von denen mindestens einige einen Durchmesser von 500 Micron oder

weniger haben, wobei mindestens einige der Teilchen mit einem Durchmesser von 500 Micron oder weniger eine erste Porendichte in einem inneren Bereich und eine zweite Porendichte in einem Oberflächenbereich haben, wobei die erste Dichte verschieden von der zweiten Dichte ist; und
ein Trägerfluid, wobei die Vielzahl von Teilchen in dem Trägerfluid sind.

**23.** Zusammensetzung nach Anspruch 22, wobei das Trägerfluid eine Salzlösung umfasst.

**24.** Zusammensetzung nach Anspruch 22, wobei das Trägerfluid ein Kontrastmittel umfasst.

**25.** Zusammensetzung nach Anspruch 22, wobei die Vielzahl von Teilchen einen mittleren Durchmesser von 500 Micron oder weniger hat.

**26.** Zusammensetzung nach Anspruch 22, wobei die Vielzahl von Teilchen einen mittleren Durchmesser von 10 Micron oder mehr hat.

**27.** Zusammensetzung nach Anspruch 22, wobei die Vielzahl von Teilchen einen mittleren Durchmesser von 100 Micron oder mehr hat.

**28.** Zusammensetzung nach Anspruch 27, wobei die Vielzahl von Teilchen einen mittleren Durchmesser von 300 Micron oder weniger hat.

**29.** Zusammensetzung nach Anspruch 22, wobei die Vielzahl von Teilchen einen mittleren Durchmesser von 300 Micron oder mehr hat.

**30.** Zusammensetzung, geeignet für Embolisation, aufweisend:

eine Vielzahl von polymeren Teilchen, von denen mindestens einige einen Durchmesser von 500 Micron oder weniger aufweisen, wobei mindestens einige der Teilchen mit einem Durchmesser von 500 Micron oder weniger eine erste durchschnittliche Porengröße in einem inneren Bereich und einen zweite durchschnittliche Porengröße in einem Oberflächenbereich haben, wobei die erste durchschnittliche Porengröße verschieden von der zweiten durchschnittlichen Porengröße ist; und
ein Trägerfluid, wobei die Vielzahl der Teilchen in dem Trägerfluid sind.

**31.** Verfahren zum Herstellen von polymeren Teilchen, wie in einem der Ansprüche 1 bis 19 definiert, umfassend:

Ausbilden von Tropfen, welche ein Basispolymer und eine Gelierungsvorstufe enthalten; und
Kontaktieren der Tropfen mit einem Gelbildner, um Teilchen zu bilden, welche das Basispolymer und die Gelierungsvorstufe enthalten, wobei der Gelbildner in einem Gefäß enthalten ist, und wobei das Verfahren weiter mindestens ein Element, ausgewählt aus der Gruppe bestehend aus Sprudeln eines Gases durch den Gelbildner, Mitverwenden eines Tensids in der den Gelbildner enthaltenden Mischung, Anordnen eines den Gelbildner enthaltenden Nebels zwischen einer Tropfenquelle und dem Gefäß und Rühren des Gelbildners enthält.

## Revendications

**1.** Particule polymère appropriée pour faire partie d'une composition pour une embolisation ayant un diamètre de 500 micromètres ou moins, la particule ayant une première densité de pores dans une région interne et une seconde densité de pores au niveau d'une région de surface, la première densité étant différente de la seconde densité.

**2.** Particule polymère selon la revendication 1, dans laquelle la première densité est supérieure à la seconde densité.

**3.** Particule polymère selon la revendication 1, la particule ayant une première taille de pore moyenne dans la région interne et une seconde taille de pore moyenne au niveau de la région de surface, la première taille de pore moyenne étant différente de la seconde taille de pore moyenne.

**4.** Particule polymère selon la revendication 3, dans laquelle la première taille de pore moyenne est supérieure à la seconde taille de pore moyenne.

**5.** Particule polymère selon la revendication 1, la particule ayant un diamètre de 10 micromètres ou plus.

**6.** Particule polymère selon la revendication 1, la particule ayant un diamètre de 100 micromètres ou plus.

**7.** Particule polymère selon la revendication 6, la particule ayant un diamètre de 300 micromètres ou plus.

**8.** Particule polymère selon la revendication 1, la particule ayant un diamètre de 300 micromètres ou plus.

**9.** Particule polymère selon la revendication 1, la particule comprenant au moins un polymère choisi dans le groupe consistant en des poly(alcools vinyliques), des poly(acides acryliques), des poly(acides méthacryliques), des poly (sulfonates de vinyle), des carboxyméthylcelluloses, des hydroxyéthylcelluloses, des celluloses substituées, des polyacrylamides, des polyéthylèneglycols, des polyamides, des polyurées, des polyuréthanes, des polyesters, des polyéthers, des polystyrènes, des polysaccharides, des poly(acides lactiques), des polyéthylènes, des polyméthylméthacrylates, des polycaprolactones, des poly(acides glycoliques) et des poly(acides lactiques-co-acides glycoliques).

**10.** Particule polymère selon la revendication 1, la particule comprenant un poly(alcool vinylique).

**11.** Particule polymère selon la revendication 1, la particule étant au moins partiellement revêtue d'un matériau bioabsorbable.

**12.** Particule polymère selon la revendication 1, la particule ayant une densité de 1,1 gramme par centimètre cube à 1,4 gramme par centimètre cube.

**13.** Particule polymère selon la revendication 1, la particule ayant une sphéricité de 0,9 ou plus.

**14.** Particule polymère selon la revendication 1, la particule ayant, après compression à environ 50 %, une sphéricité de 0,9 ou plus.

**15.** Particule polymère selon la revendication 1, la particule comprenant 2,5 pour cent en poids ou moins de polysaccharide.

**16.** Particule polymère selon la revendication 15, dans laquelle le polysaccharide comprend de l'alginate.

**17.** Particule polymère selon la revendication 16, dans laquelle l'alginate a une teneur en acide guluronique de 60 pour cent ou plus.

**18.** Particule polymère selon la revendication 1, la particule étant insoluble dans du DSMO.

**19.** Particule polymère selon la revendication 1, la particule étant sensiblement exempte de composés dérivés d'un animal.

**20.** Particule polymère appropriée pour faire partie d'une composition pour une embolisation ayant un diamètre de 500 micromètres ou moins, la particule ayant une première taille de pore moyenne dans une région interne et une seconde taille de pore moyenne au niveau d'une région de surface, la première taille de pore moyenne étant différente de la seconde taille de pore moyenne.

**21.** Particule polymère selon la revendication 20, dans laquelle la première taille de pore moyenne est supérieure à la seconde taille de pore moyenne.

**22.** Composition appropriée pour une embolisation comprenant :

une pluralité de particules polymères, au moins certaines de la pluralité de particules ayant un diamètre de 500 micromètres ou moins, dans laquelle au moins certaines des particules ayant un diamètre de 500 micromètres ou moins ont une première densité de pores dans une région interne et une seconde densité de pores au niveau d'une région de surface, la première densité étant différente de la seconde densité ; et
un fluide support, la pluralité de particules étant dans le fluide support.

**23.** Composition selon la revendication 22, dans laquelle le fluide support comprend une solution salée.

**24.** Composition selon la revendication 22, dans laquelle le fluide support comprend un agent de contraste.

**25.** Composition selon la revendication 22, dans laquelle la pluralité de particules a un diamètre moyen de 500 micromètres ou moins.

**26.** Composition selon la revendication 22, dans laquelle la pluralité de particules a un diamètre moyen de 10 micromètres ou plus.

**27.** Composition selon la revendication 22, dans laquelle la pluralité de particules a un diamètre moyen de 100 micromètres ou plus.

**28.** Composition selon la revendication 27, dans laquelle la pluralité de particules a un diamètre moyen de 300 micromètres ou moins.

**29.** Composition selon la revendication 22, dans laquelle la pluralité de particules a un diamètre moyen de 300 micromètres ou plus.

**30.** Composition appropriée pour une embolisation comprenant :

une pluralité de particules polymères, au moins certaines de la pluralité de particules ayant un diamètre de 500 micromètres ou moins, dans laquelle au moins certaines des particules ayant un diamètre de 500 micromètres ou moins ont une première taille de pore moyenne dans une région interne et une seconde taille de pore moyenne au niveau d'une région de surface, la première taille de pore moyenne étant différente de la seconde taille de pore moyenne ; et
un fluide support, la pluralité de particules étant donné que le fluide support.

**31.** Procédé de préparation de particules polymères selon l'une quelconque des revendications 1 à 19, comprenant :

la formation de gouttelettes contenant un polymère basique et un précurseur de gélification ; et
la mise en contact des gouttelettes avec un agent gélifiant pour former des particules contenant le polymère basique et le précurseur de gélification, l'agent gélifiant étant contenu dans un récipient, et le procédé comprenant en outre au moins un membre choisi dans le groupe consistant en le barbotage d'un gaz dans l'agent gélifiant, comprenant un agent tensioactif dans le mélange contenant l'agent gélifiant, le dépôt d'un brouillard contenant l'agent gélifiant entre une source des gouttelettes et le récipient, et l'agitation de l'agent gélifiant.

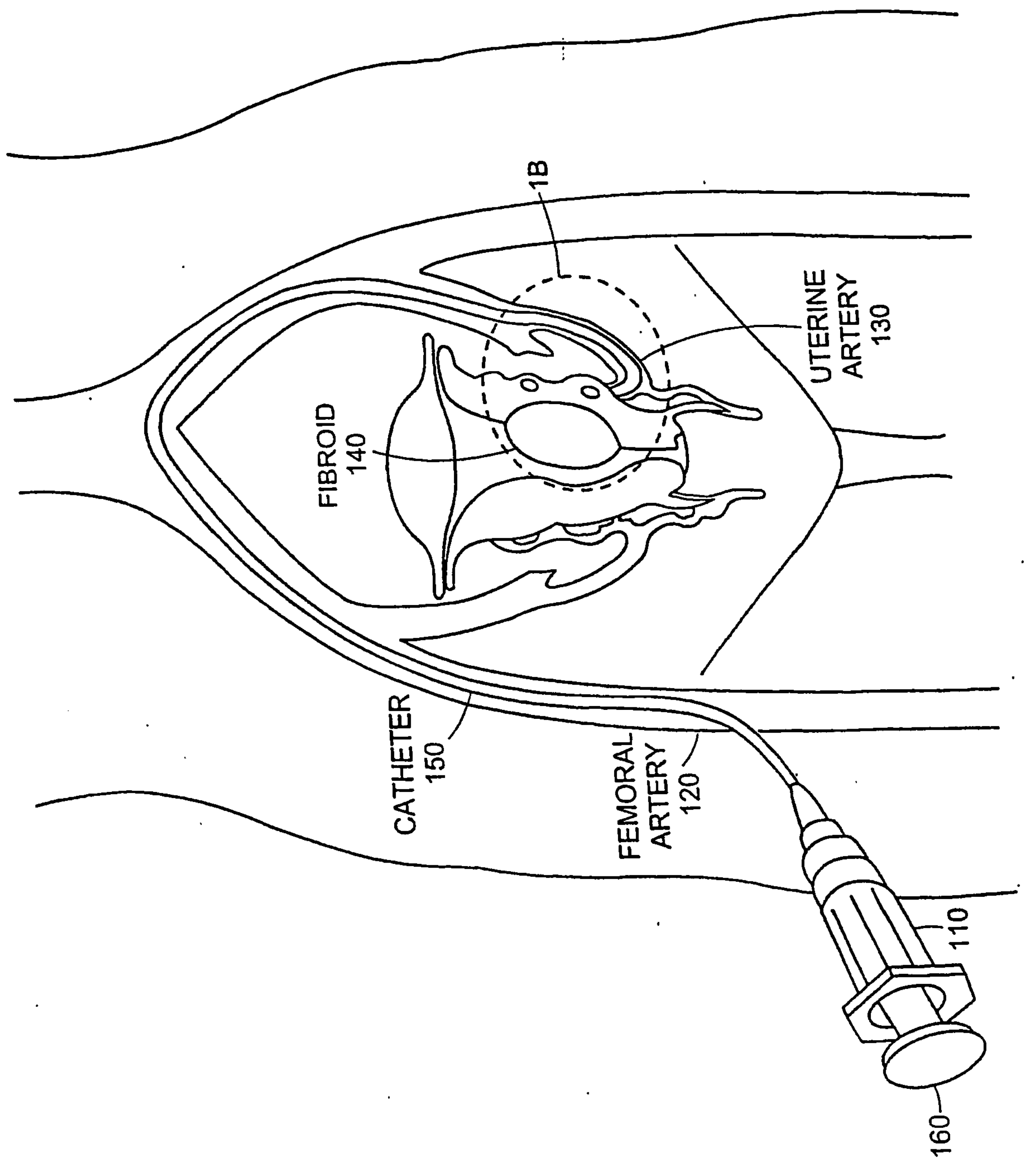
1B
UTERINE ARTERY 130
FIBROID 140
CATHETER 150
FEMORAL ARTERY 120
110
160

FIG. 1A

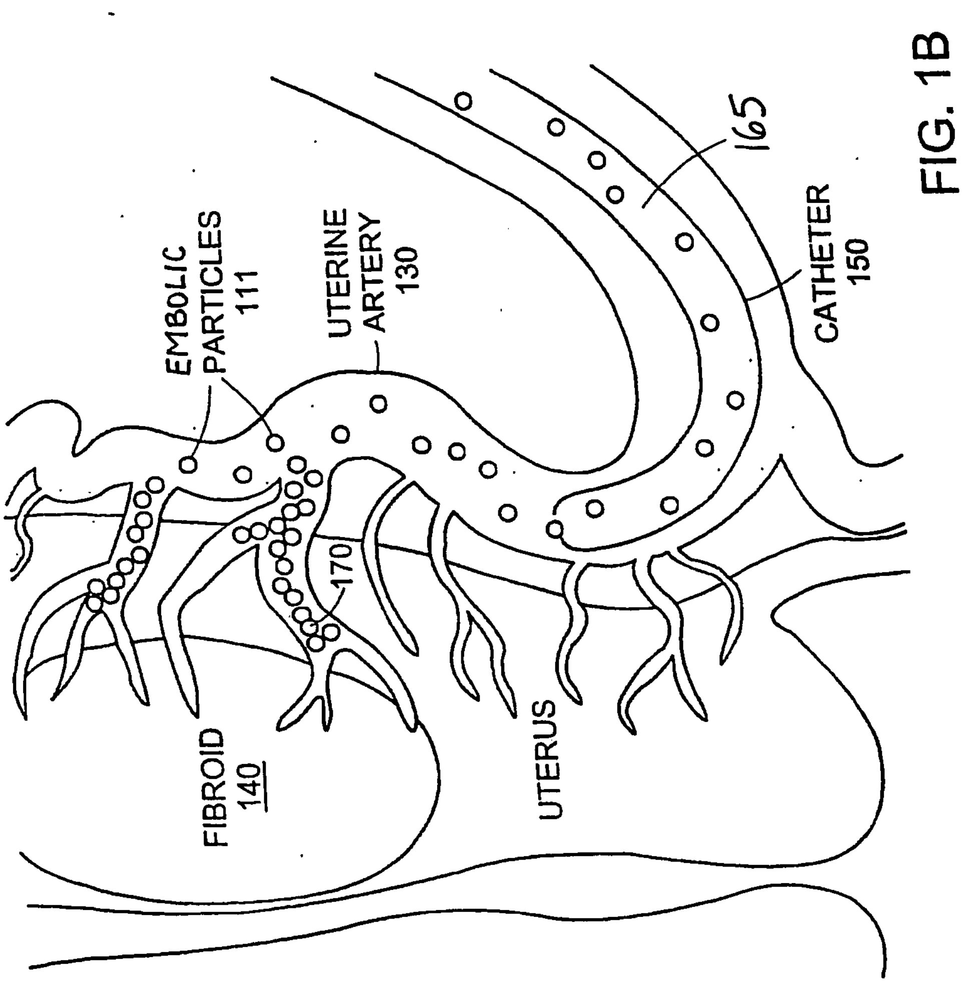
EMBOLIC PARTICLES 111
UTERINE ARTERY 130
165
CATHETER 150
170
FIBROID 140
UTERUS

FIG. 1B

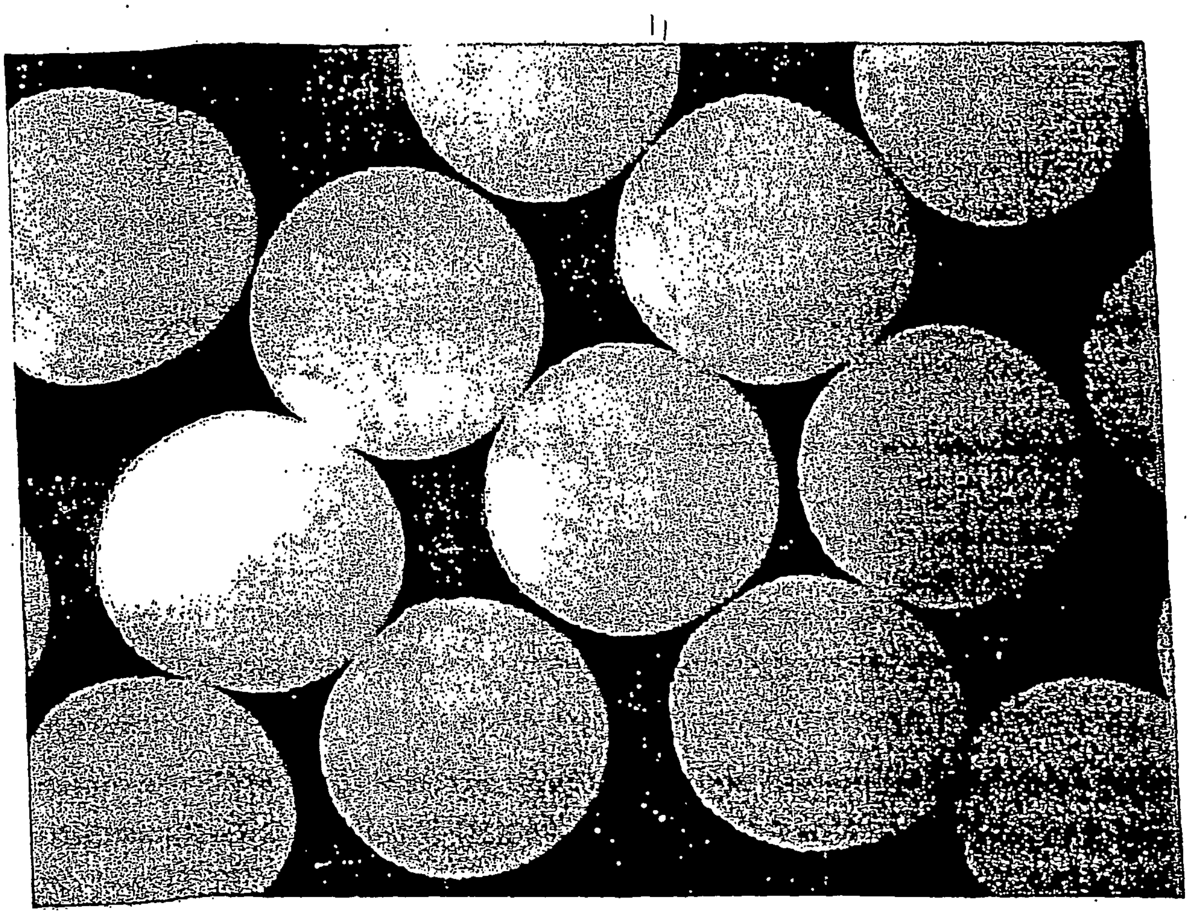

FIG. 2A

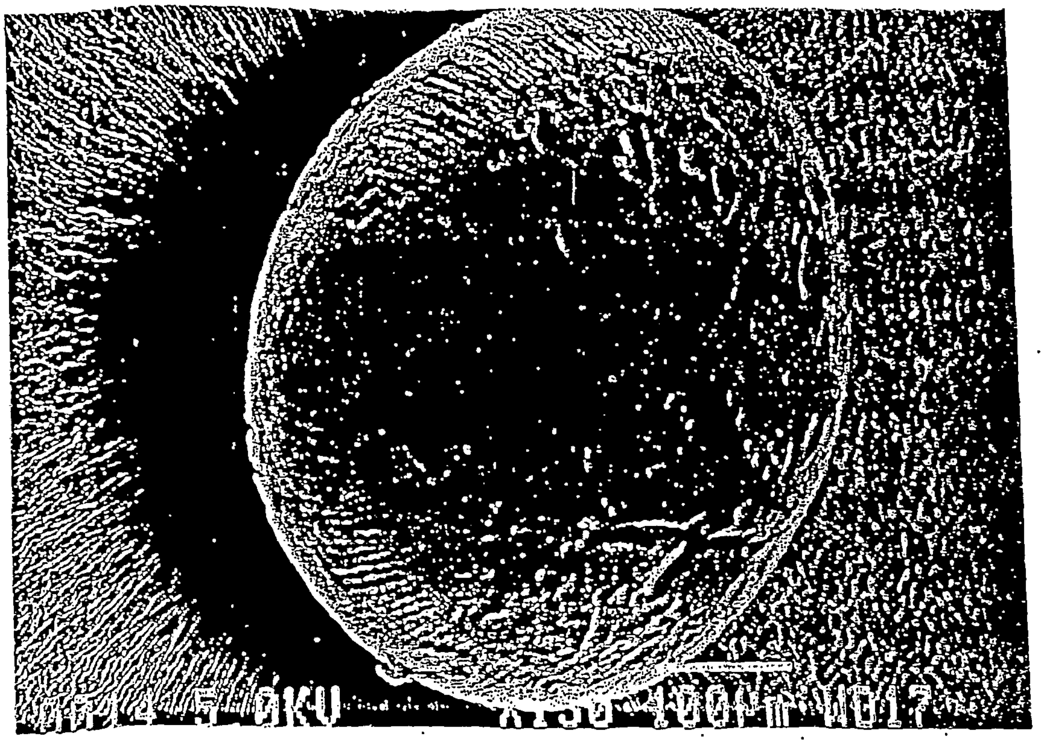

FIG. 2B

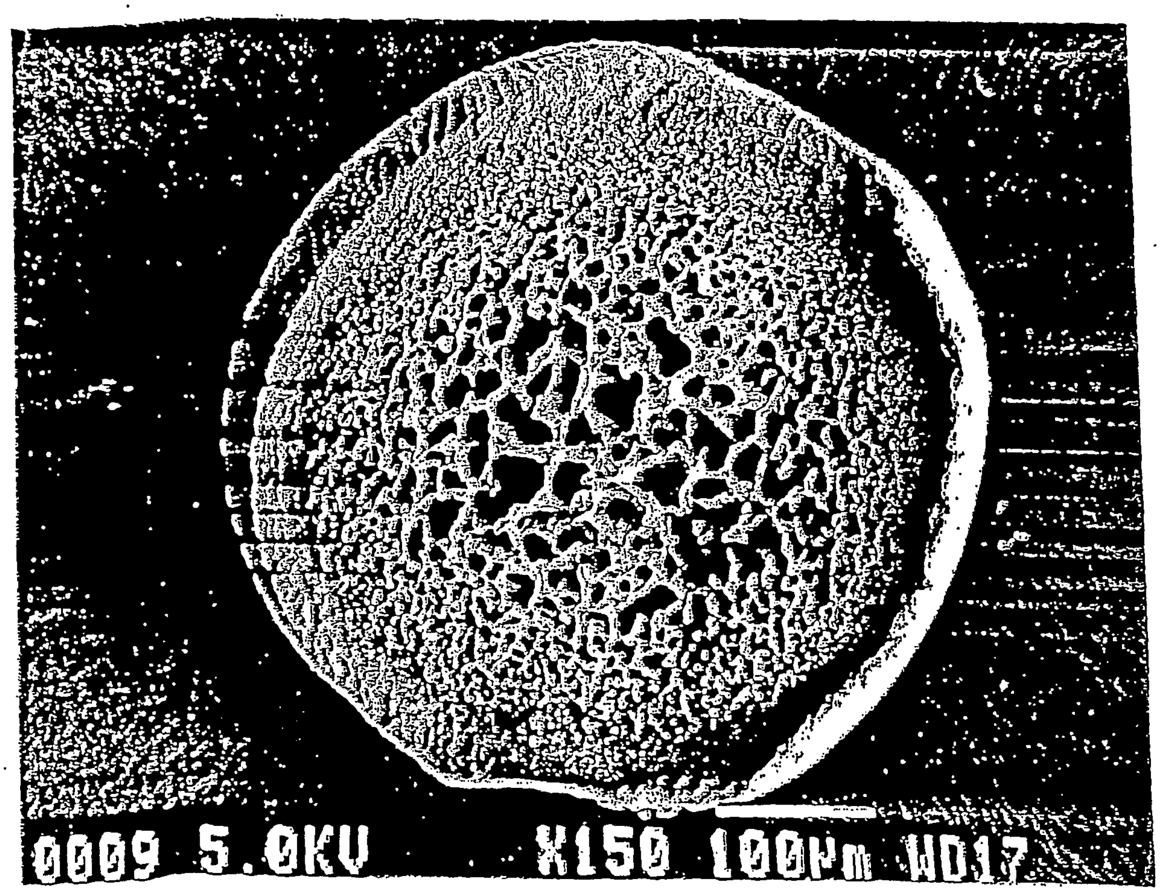


FIG. 2C

r 2r/3 r/3 c′

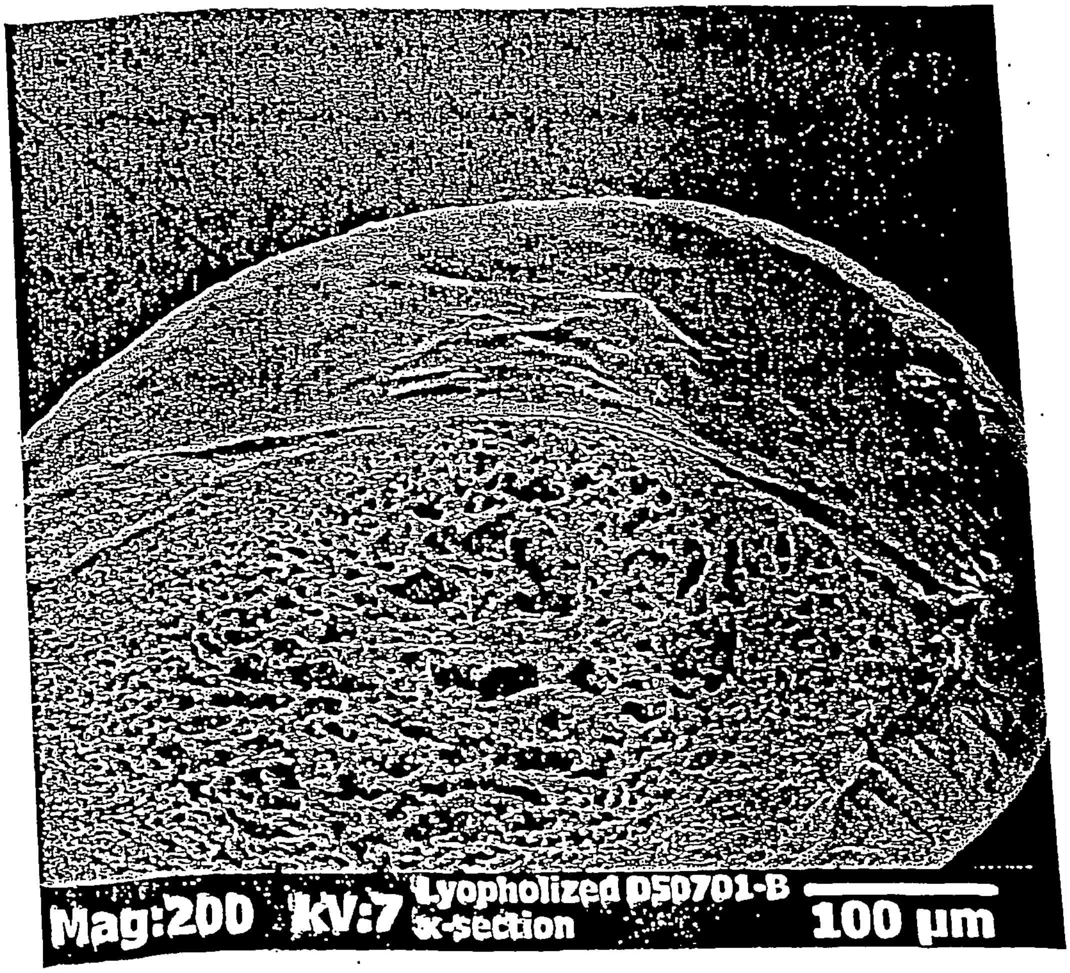


FIG. 2D

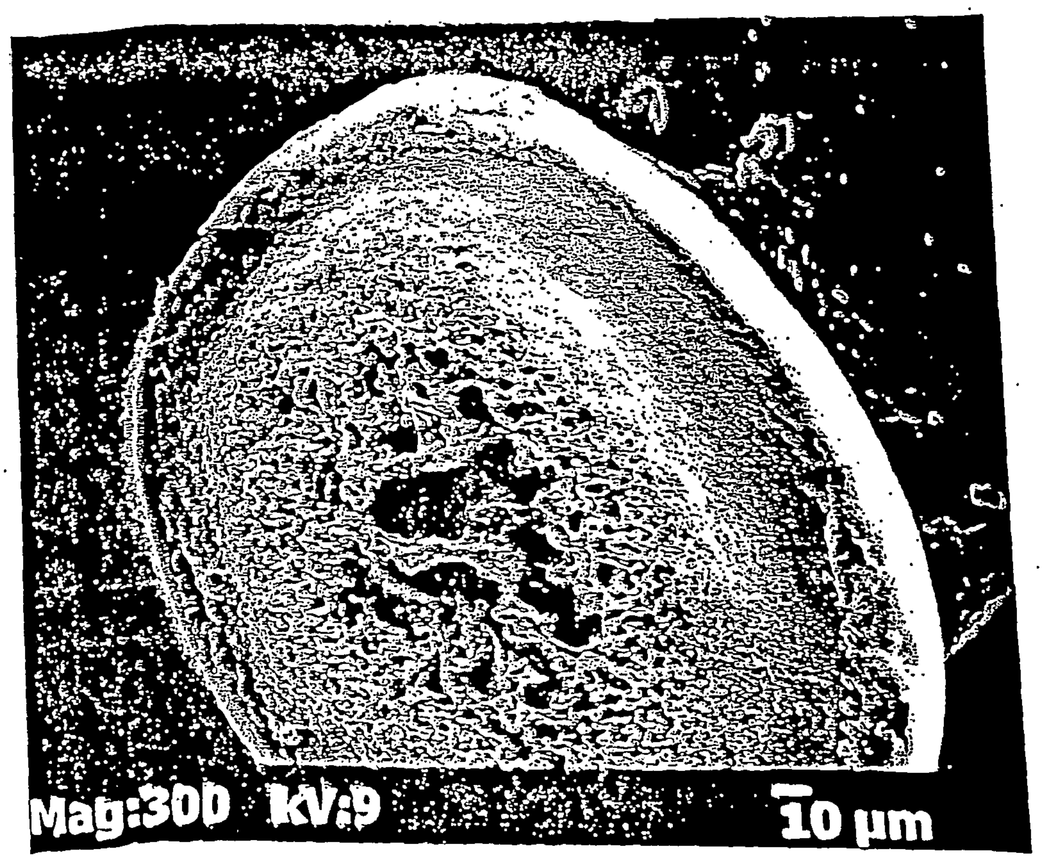


FIG. 2E

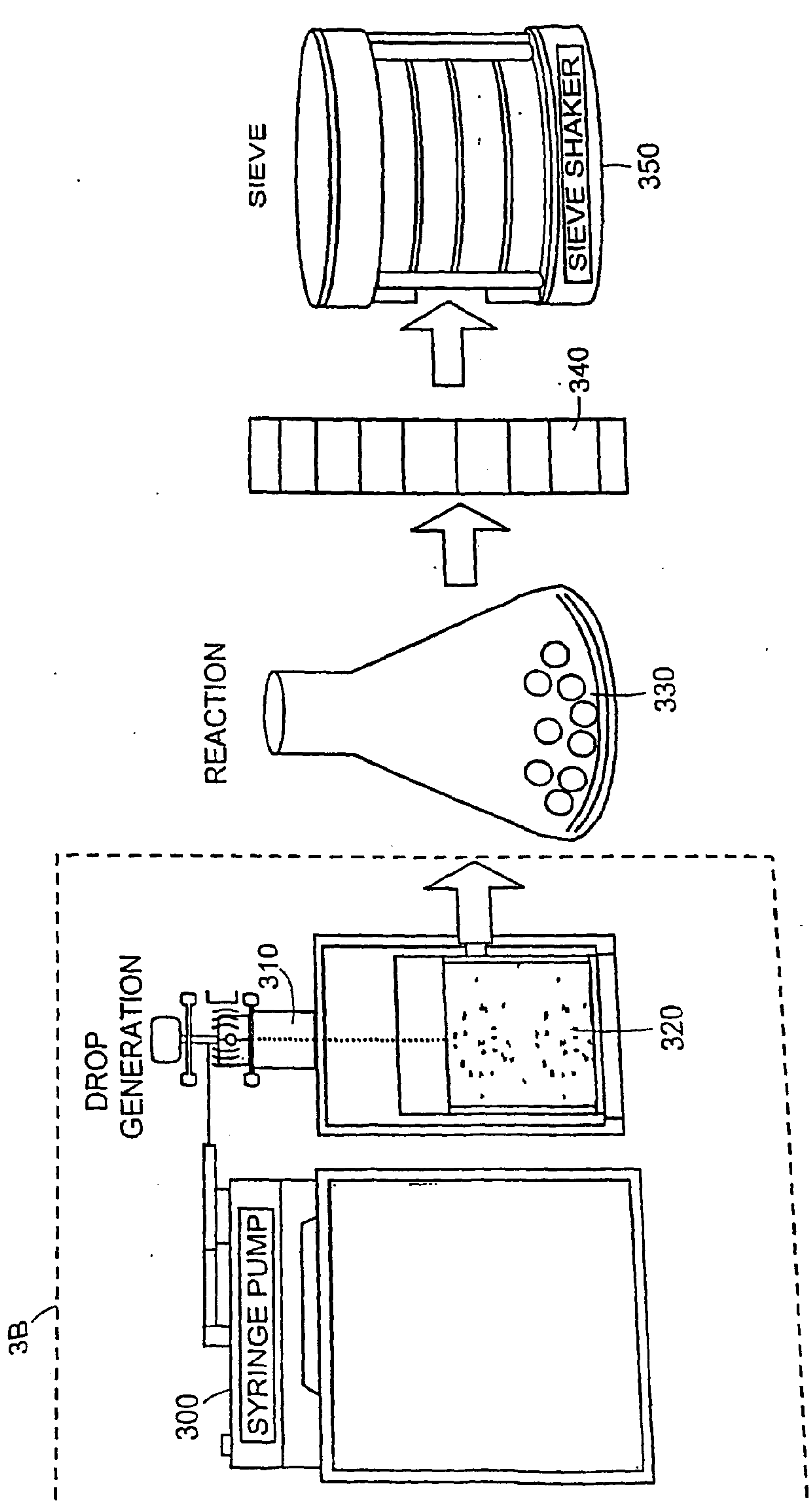
SIEVE
SIEVE SHAKER
350
340
REACTION
330
DROP
GENERATION
310
320
SYRINGE PUMP
300
3B

FIG. 3A

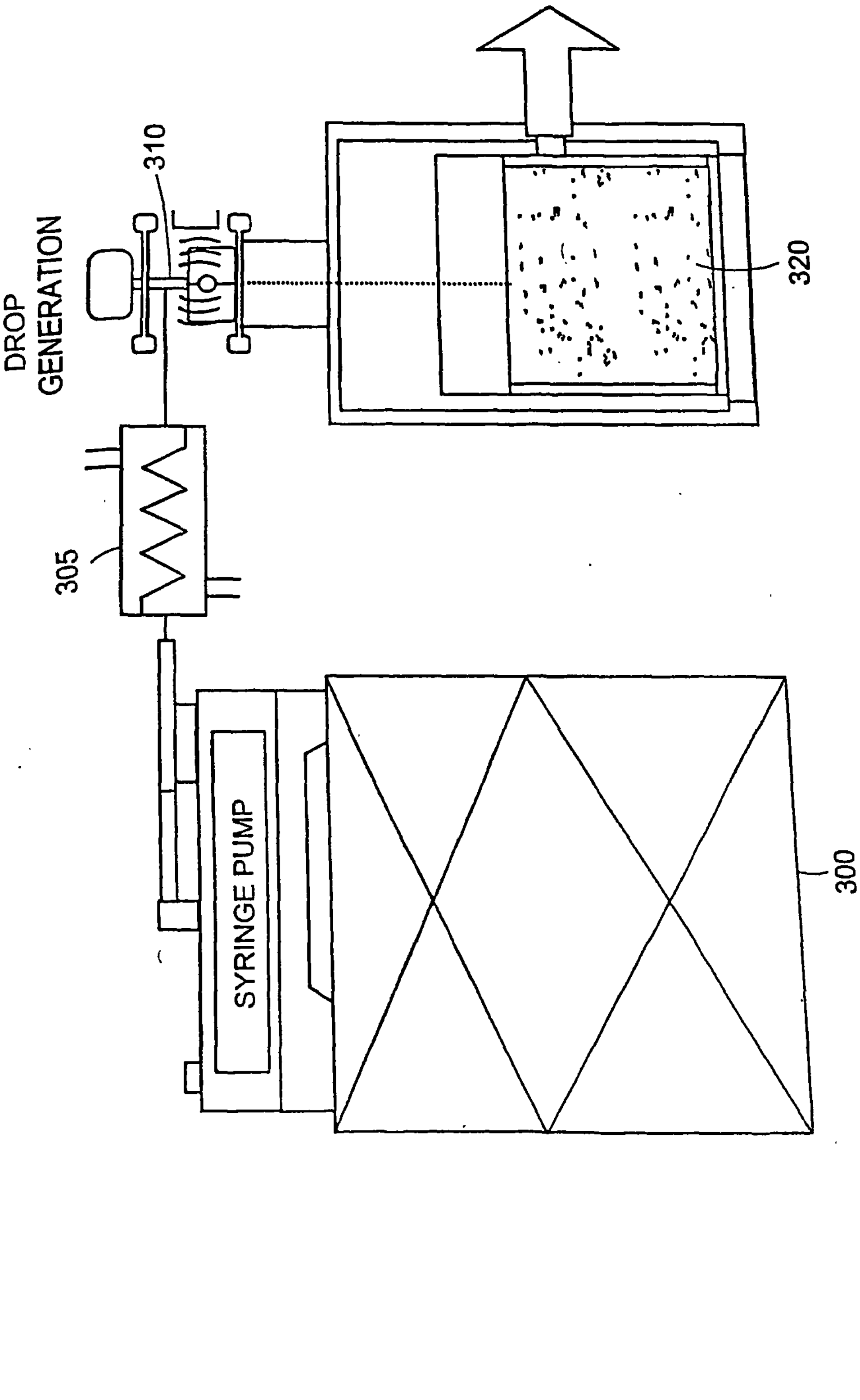


FIG. 3B

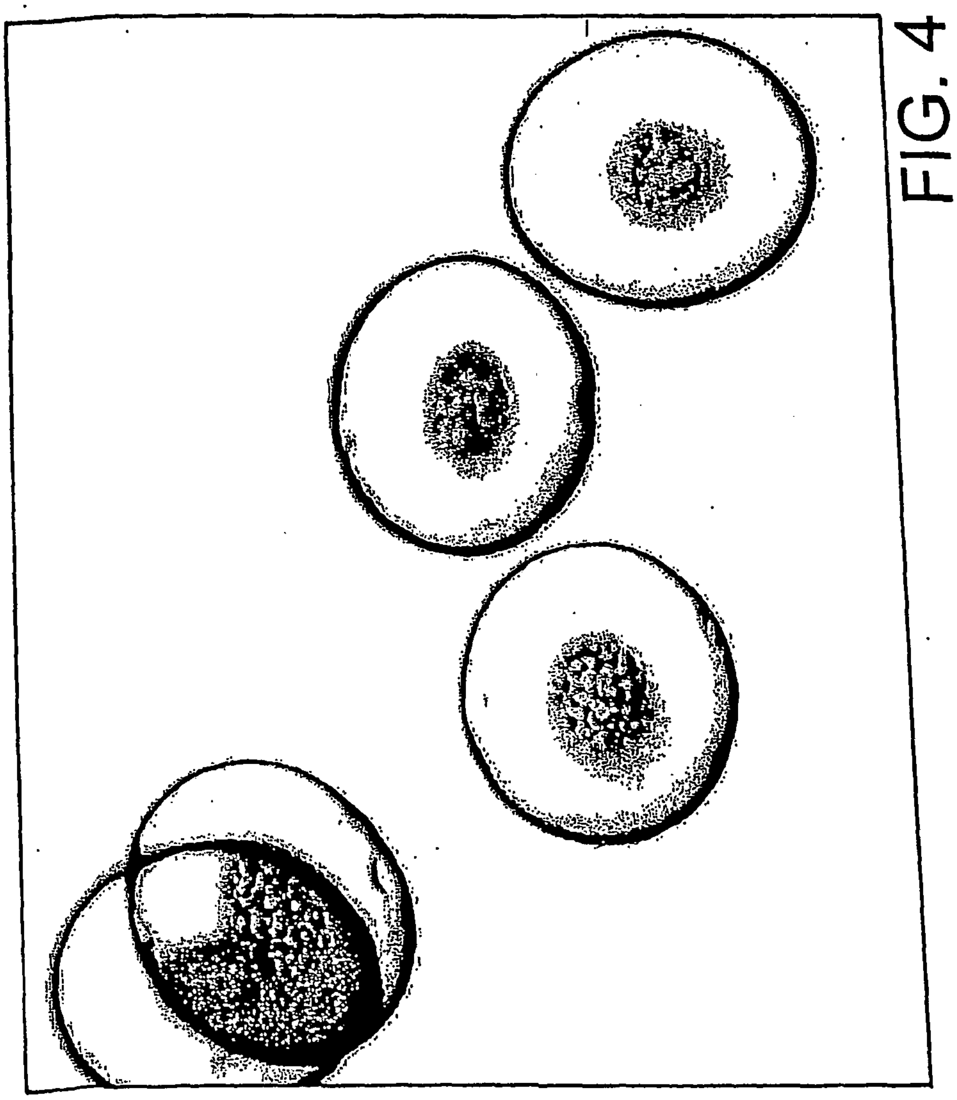

FIG. 4

ECA DIAMETER DIFFERENTIAL VOLUME
600.0 - 1400.0 MICRONS

| | |
|---|---|
| TOTAL COUNT | 958 |
| MEAN | 810.7 MICRONS |
| STANDARD DEVIATION | 102.4 MICRONS |
| COEFFICIENT OF VARIANCE | 12.63% |
| HARMONIC MEAN | 800.4 MICRONS |
| MODE | 783.6 MICRONS |
| SKEWNESS | 2.26 |
| 10% | 730.2 MICRONS |
| 25% | 755.2 MICRONS |
| 50% | 785.8 MICRONS |
| 75% | 816.4 MICRONS |
| 90% | 954.1 MICRONS |
| PERCENT OF TOTAL | 100.00% |

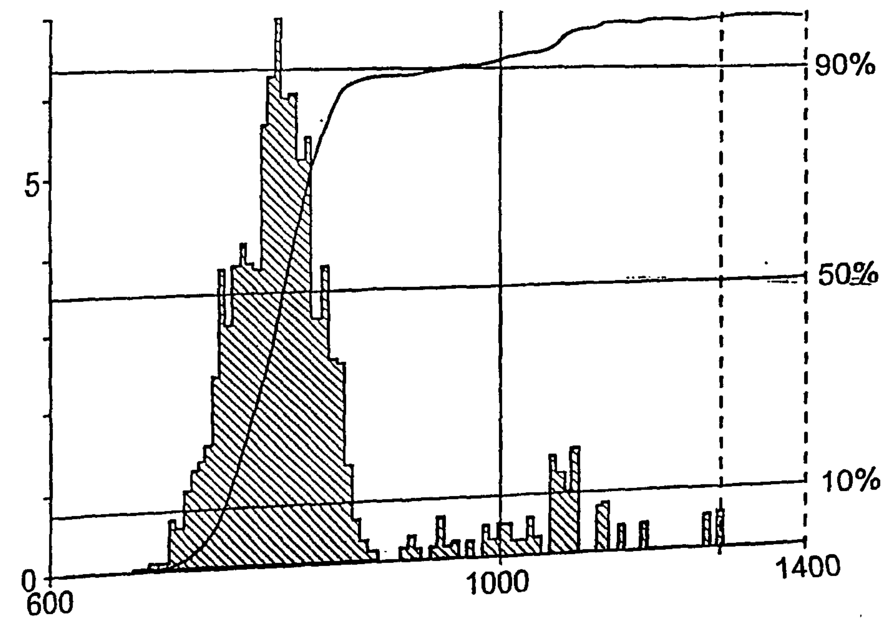


FIG. 5

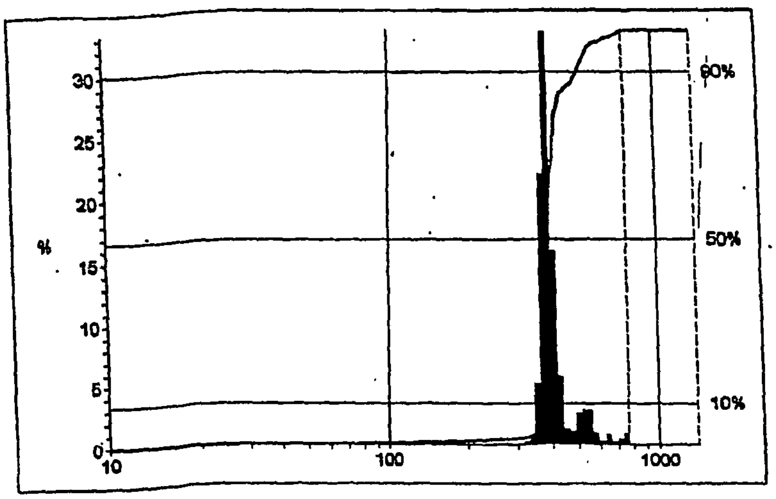


FIG. 6

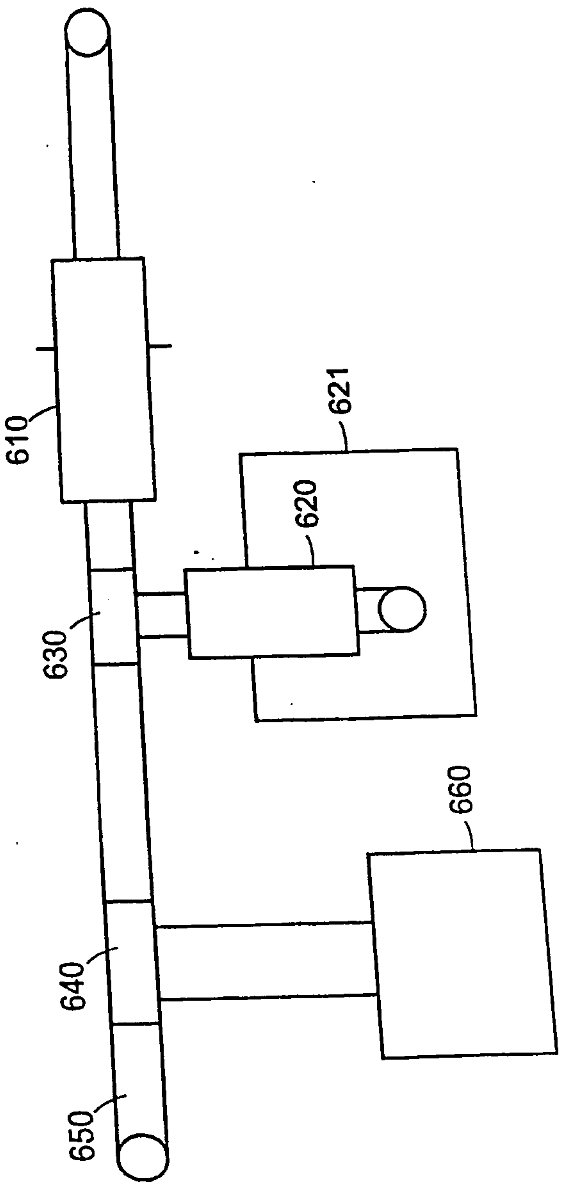


FIG. 7

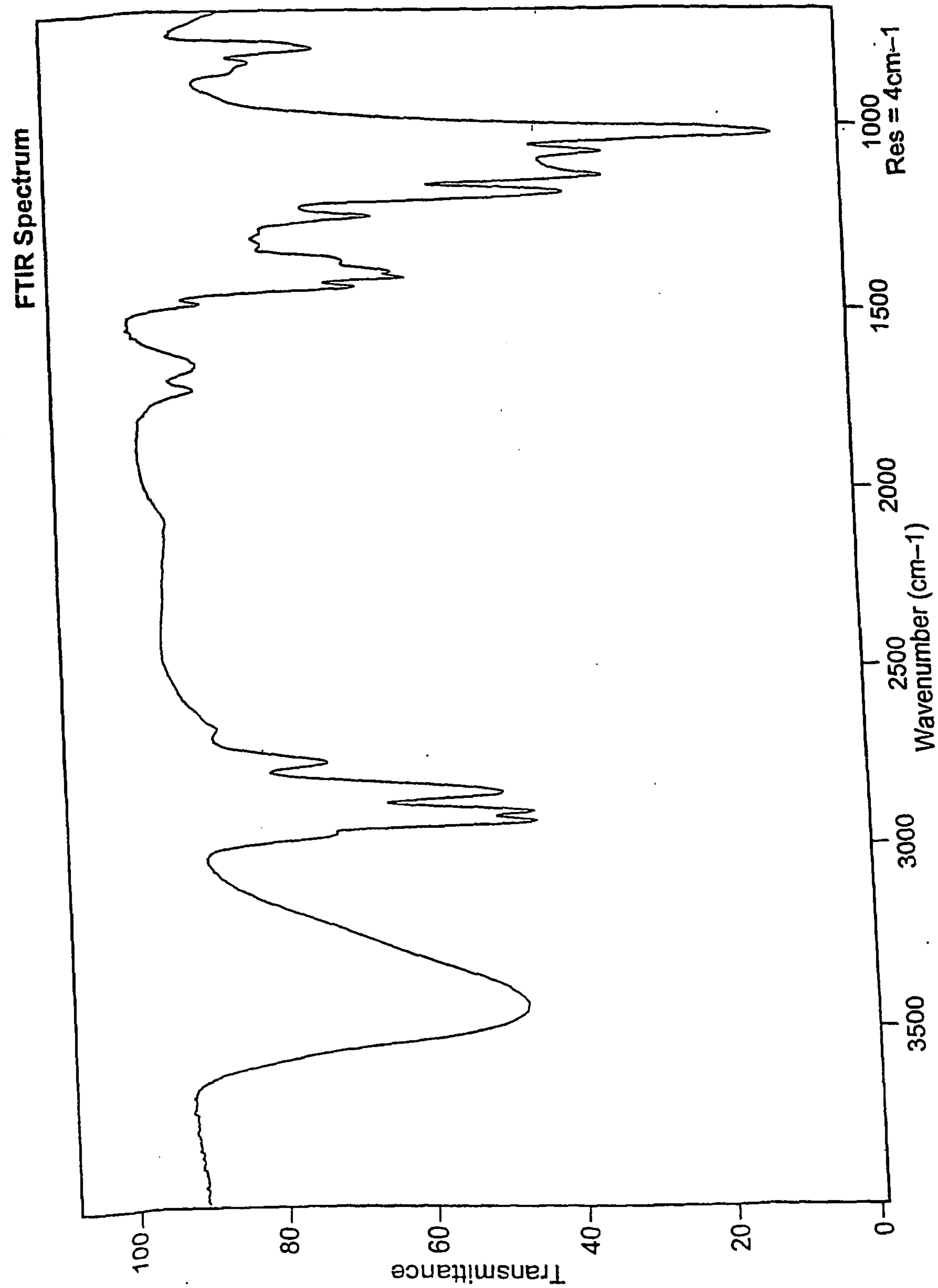
FTIR Spectrum
Transmittance
100
80
60
40
20
0
3500
3000
2500
2000
1500
1000
Wavenumber (cm−1)
Res = 4cm−1

FIG. 8

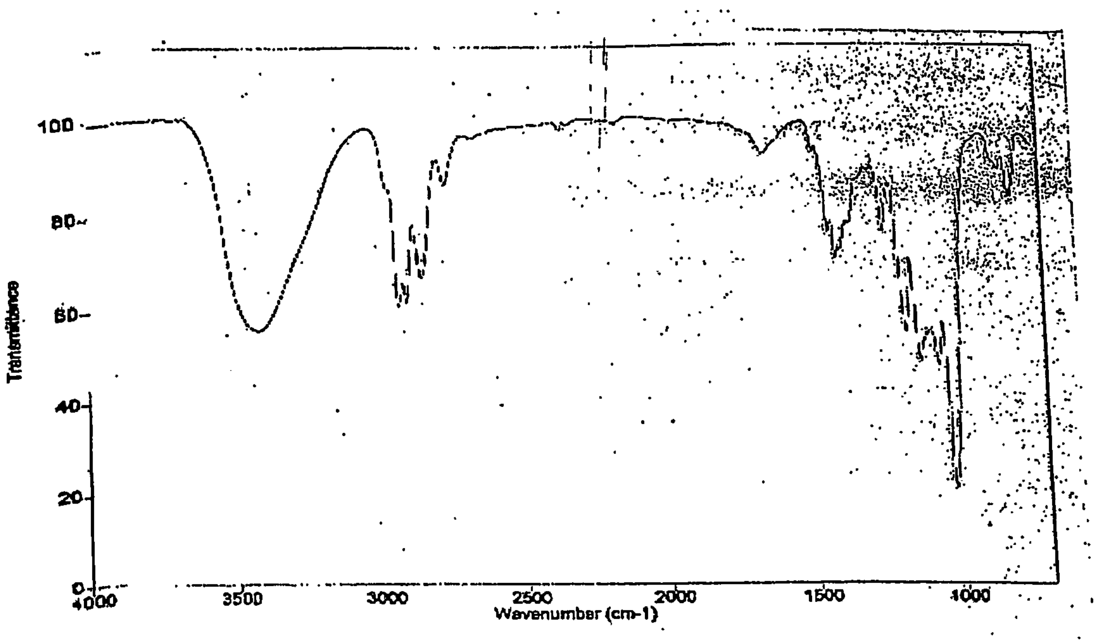
100
80
60
40
20
0
Transmittance
4000
3500
3000
2500
2000
1500
1000
Wavenumber (cm-1)

FIG. 9